# EUROPEAN PATENT APPLICATION

(11) **EP 1 808 443 A1**
(43) Date of publication of application: **18.07.2007**
(21) Application number: 06290092.3
(22) Date of filing: 13.01.2006
(51) Int. Cl.: C07K 14/445, A61K 39/015, A61P 33/06

(54) **Purified polypeptide comprising or consisting of a C-terminus MSP1 antigen from Plasmodium falciparum carrying a Glycosyl-phosphatidyl-inositol group (GPI)**

(71) Applicant: INSTITUT PASTEUR, 75724 Paris Cédex 15 (FR); CENTRE NATIONAL DE LA RECHERCHE SCIENTIFIQUE, 75794 Paris Cedex 16 (FR)
(72) Inventor: Longacre, Shirley, 75006 Paris (FR); Bonnet, Sarah, 44300 Nantes (FR); Fontaine, Thierry, 92140 Clamart (FR)
(74) Representative: Desaix, Anne

(57) **Abstract**

The present invention relates to a purified polypeptide comprising or consisting of a C-terminus MSP1 antigen from *Plasmodium falciparum* carrying a Glycosyl-phosphatidyl-inositol group (GPI), an immunogenic composition and a vaccine against a *Plasmodium* infection comprising said purified polypeptide.

SEQUENCE LISTING

<110>  INSITUT PASTEUR
       CENTRE NATIONAL DE LA RECHERCHE SCIENTIFIQUE

<120>  PURIFIED POLYPEPTIDE COMPRISING OR CONSISTING OF A C-TERMINUS
       MSP1 ANTIGEN FROM PLASMODIUM FALCIMPARUM CARRYING A
       GLYCOSYL-PHOSPHATIDYL-INOSITOL GROUP (GPI)

<130>  B6211 - AD/YH/KP

<160>  23

<170>  PatentIn version 3.3

<210>  1
<211>  140
<212>  PRT
<213>  Plasmodium falciparum

<400>  1

Val Thr His Glu Ser Tyr Gln Glu Leu Val Lys Lys Leu Glu Ala Phe
1               5                   10                  15

Glu Asp Ala Val Leu Thr Gly Tyr Glu Phe Asn Thr Ile Ile Ser Lys
            20                  25                  30

Leu Ile Glu Gly Lys Phe Gln Asp Met Leu Asn Ile Ser Gln His Gln
        35                  40                  45

Cys Val Lys Lys Gln Cys Pro Glu Asn Ser Gly Cys Phe Arg His Leu
    50                  55                  60

Asp Glu Arg Glu Glu Cys Lys Cys Leu Leu Asn Tyr Lys Gln Glu Gly
65                  70                  75                  80

Asp Lys Cys Val Glu Asn Pro Asn Pro Thr Cys Asn Glu Asn Asn Gly
                85                  90                  95

Gly Cys Asp Ala Asp Ala Lys Cys Thr Glu Glu Asp Ser Gly Ser Asn
            100                 105                 110

Gly Lys Lys Ile Thr Cys Glu Cys Thr Lys Pro Asp Ser Tyr Pro Leu
        115                 120                 125

Phe Asp Gly Ile Phe Cys Ser Gly Ile Phe Ser Ser
    130                 135                 140

<210>  2
<211>  146
<212>  PRT
<213>  Artificial

<220>
<223>  MSP1-19/His

<400>  2

Val Thr His Glu Ser Tyr Gln Glu Leu Val Lys Lys Leu Glu Ala Phe
1               5                   10                  15

Glu Asp Ala Val Leu Thr Gly Tyr Glu Phe Asn Thr Ile Ile Ser Lys
            20                  25                  30

Leu Ile Glu Gly Lys Phe Gln Asp Met Leu Asn Ile Ser Gln His Gln
        35                  40                  45

Cys Val Lys Lys Gln Cys Pro Glu Asn Ser Gly Cys Phe Arg His Leu
    50                  55                  60

Asp Glu Arg Glu Glu Cys Lys Cys Leu Leu Asn Tyr Lys Gln Glu Gly
65                  70                  75                  80

Asp Lys Cys Val Glu Asn Pro Asn Pro Thr Cys Asn Glu Asn Asn Gly
                85                  90                  95

Gly Cys Asp Ala Asp Ala Lys Cys Thr Glu Glu Asp Ser Gly Ser Asn
            100                 105                 110

Gly Lys Lys Ile Thr Cys Glu Cys Thr Lys Pro Asp Ser Tyr Pro Leu
        115                 120                 125

Phe Asp Gly Ile Phe Cys Ser His His His His His His Gly Ile Phe
    130                 135                 140

Ser Ser
145

<210>  3
<211>  19
<212>  PRT
<213>  Artificial

<220>
<223>  Signal Peptide

<400>  3

Met Lys Ile Ile Phe Phe Leu Cys Ser Phe Leu Phe Phe Ile Ile Asn
1               5                   10                  15

Thr Gln Cys

<210>  4
<211>  21
<212>  PRT
<213>  Artificial

<220>
<223>  Anchor peptide

<400>  4

Ser Ser Asn Phe Leu Gly Ile Ser Phe Leu Leu Ile Leu Met Leu Ile
1               5                   10                  15

Leu Tyr Ser Phe Ile
            20

<210>  5
<211>  564
<212>  DNA
<213>  Artificial

<220>
<223>  PfMSP1p19/His/GPI

<220>
<221>  CDS
<222>  (1)..(564)

<400>  5
atg aag atc ata ttc ttt ttg tgt tcg ttc ctc ttc ttc atc atc aac      48
Met Lys Ile Ile Phe Phe Leu Cys Ser Phe Leu Phe Phe Ile Ile Asn
1               5                   10                  15

acg caa tgt gtg acc cac gag agc tac caa gag ctc gtc aag aaa ctg      96
Thr Gln Cys Val Thr His Glu Ser Tyr Gln Glu Leu Val Lys Lys Leu
            20                  25                  30

gag gcc ttc gag gac gcg gtg ttg acc ggc tac gaa ttc aac acg atc      144
Glu Ala Phe Glu Asp Ala Val Leu Thr Gly Tyr Glu Phe Asn Thr Ile
        35                  40                  45

atc tcg aaa ttg atc gag ggc aaa ttc caa gac atg ttg aac atc tcg      192
Ile Ser Lys Leu Ile Glu Gly Lys Phe Gln Asp Met Leu Asn Ile Ser
    50                  55                  60

cag cac caa tgc gtg aaa aaa caa tgt ccc gag aac tct ggc tgt ttc      240
Gln His Gln Cys Val Lys Lys Gln Cys Pro Glu Asn Ser Gly Cys Phe
65                  70                  75                  80

aga cac ttg gac gag aga gag gag tgt aaa tgt ctg ctg aac tac aaa      288
Arg His Leu Asp Glu Arg Glu Glu Cys Lys Cys Leu Leu Asn Tyr Lys
                85                  90                  95

cag gag ggc gac aag tgc gtg gag aac ccc aac ccg acc tgt aac gag      336
Gln Glu Gly Asp Lys Cys Val Glu Asn Pro Asn Pro Thr Cys Asn Glu
            100                 105                 110

aac aac ggc ggc tgt gac gca gac gcc aaa tgc acc gag gag gac tcg      384
Asn Asn Gly Gly Cys Asp Ala Asp Ala Lys Cys Thr Glu Glu Asp Ser
        115                 120                 125

ggc agc aac ggc aag aaa atc acg tgt gag tgt acc aaa ccc gac tcg      432
Gly Ser Asn Gly Lys Lys Ile Thr Cys Glu Cys Thr Lys Pro Asp Ser
    130                 135                 140

tac ccg ctg ttc gac ggc atc ttc tgc agc cac cat cac cat cac cat      480
Tyr Pro Leu Phe Asp Gly Ile Phe Cys Ser His His His His His His
145                 150                 155                 160

ggc atc ttc agc agc tcc tct aac ttc ttg ggc atc tcg ttc ttg ttg      528
Gly Ile Phe Ser Ser Ser Ser Asn Phe Leu Gly Ile Ser Phe Leu Leu
                165                 170                 175

atc ctc atg ttg atc ttg tac agc ttc att taa taa                      564
Ile Leu Met Leu Ile Leu Tyr Ser Phe Ile

180                 185

<210>  6
<211>  186
<212>  PRT
<213>  Artificial

<220>
<223>  Synthetic Construct

<400>  6

Met Lys Ile Ile Phe Phe Leu Cys Ser Phe Leu Phe Phe Ile Ile Asn
1               5                   10                  15

Thr Gln Cys Val Thr His Glu Ser Tyr Gln Glu Leu Val Lys Lys Leu
            20                  25                  30

Glu Ala Phe Glu Asp Ala Val Leu Thr Gly Tyr Glu Phe Asn Thr Ile
        35                  40                  45

Ile Ser Lys Leu Ile Glu Gly Lys Phe Gln Asp Met Leu Asn Ile Ser
    50                  55                  60

Gln His Gln Cys Val Lys Lys Gln Cys Pro Glu Asn Ser Gly Cys Phe
65                  70                  75                  80

Arg His Leu Asp Glu Arg Glu Glu Cys Lys Cys Leu Leu Asn Tyr Lys
                85                  90                  95

Gln Glu Gly Asp Lys Cys Val Glu Asn Pro Asn Pro Thr Cys Asn Glu
            100                 105                 110

Asn Asn Gly Gly Cys Asp Ala Asp Ala Lys Cys Thr Glu Glu Asp Ser
        115                 120                 125

Gly Ser Asn Gly Lys Lys Ile Thr Cys Glu Cys Thr Lys Pro Asp Ser
    130                 135                 140

Tyr Pro Leu Phe Asp Gly Ile Phe Cys Ser His His His His His His
145                 150                 155                 160

Gly Ile Phe Ser Ser Ser Ser Asn Phe Leu Gly Ile Ser Phe Leu Leu
                165                 170                 175

Ile Leu Met Leu Ile Leu Tyr Ser Phe Ile
            180                 185

<210>  7
<211>  49
<212>  DNA
<213>  Artificial

<220>
<223>  Sig 1

<400>  7
cccggatccg aaaatgaaga tcatattctt tttgtgttcg ttcctcttc                    49


<210>  8
<211>  48
<212>  DNA
<213>  Artificial

<220>
<223>  Sig 2

<400>  8
tctcgtgggt cacacattgc gtgttgatga tgaagaagag gaacgaac                     48


<210>  9
<211>  50
<212>  DNA
<213>  Artificial

<220>
<223>  Sig 3

<400>  9
gtgtgaccca cgagagctac caagagctcg tcaagaaact ggaggccttc                   50


<210>  10
<211>  50
<212>  DNA
<213>  Artificial

<220>
<223>  Sig 4

<400>  10
cccgaattcg tagccggtca acaccgcgtc ctcgaaaggc ctccagtttc                   50


<210>  11
<211>  84
<212>  DNA
<213>  Artificial

<220>
<223>  Bac 1

<400>  11
ggggaattca acatctcgca gcaccaatgc gtgaaaaaac aatgtcccga gaactctggc        60

tgtttcagac acttggacga gaga                                               84


<210>  12
<211>  87
<212>  DNA
<213>  Artificial

<220>
<223>  Bac 2

<400>  12
acaggtcggg ttggggttct ccacgcactt gtcgccctcc tgtttgtagt tcagcagaca        60

tttacactcc tctctctcgt ccaagtg                                            87

<210>  13
<211>  84
<212>  DNA
<213>  Artificial

<220>
<223>  Bac 3

<400>  13
cccaacccga cctgtaacga gaacaacggc ggctgtgacg ccgacgccaa atgcaccgag      60

gaggactcgg gcagcaacgg caag                                              84


<210>  14
<211>  84
<212>  DNA
<213>  Artificial

<220>
<223>  Bac 4

<400>  14
agaggagctg cagaagatgc cgtcgaacag cgggtacgag tcgggtttgg tacactcaca      60

cgtgattttc ttgccgttgc tgcc                                              84


<210>  15
<211>  87
<212>  DNA
<213>  Artificial

<220>
<223>  Bac 5

<400>  15
ttctgcagct cctctaactt cttgggcatc tcgttcttgt tgatcctcat gttgatcttg      60

tacagcttca tttaataaag atctccc                                           87


<210>  16
<211>  23
<212>  DNA
<213>  Artificial

<220>
<223>  Bac 6

<400>  16
gggagatctt tattaaatga agc                                               23


<210>  17
<211>  22
<212>  DNA
<213>  Artificial

<220>
<223>  Bac 7

<400>  17
ggggaattca acatctcgca gc                                                22


<210>  18

<211>  45
<212>  DNA
<213>  Artificial

<220>
<223>  Bac 8

<400>  18
gcccaaagat ctttattagc tgcagaagat gccgtcgaac agcgg                    45


<210>  19
<211>  516
<212>  DNA
<213>  Artificial

<220>
<223>  PvMSP1p19/His/GPI


<220>
<221>  CDS
<222>  (1)..(516)

<400>  19
atg aag gcg cta ctc ttt ttg ttc tct ttc att ttt ttc gtt acc aaa      48
Met Lys Ala Leu Leu Phe Leu Phe Ser Phe Ile Phe Phe Val Thr Lys
1               5                   10                  15

tgt caa tgt gaa aca gaa agt tat aag cag ctt gta gcc aac gtg gac      96
Cys Gln Cys Glu Thr Glu Ser Tyr Lys Gln Leu Val Ala Asn Val Asp
            20                  25                  30

gaa ttc ata tta tcc cag ctg cta aat gtg caa act cag tta tta act      144
Glu Phe Ile Leu Ser Gln Leu Leu Asn Val Gln Thr Gln Leu Leu Thr
        35                  40                  45

atg agc tcc gag cac aca tgt ata gac acc aat gtg cct gat aat gca      192
Met Ser Ser Glu His Thr Cys Ile Asp Thr Asn Val Pro Asp Asn Ala
    50                  55                  60

gcc tgc tat agg tac ttg gac gga acg gaa gaa tgg aga tgc ttg tta      240
Ala Cys Tyr Arg Tyr Leu Asp Gly Thr Glu Glu Trp Arg Cys Leu Leu
65                  70                  75                  80

acc ttt aaa gaa gaa ggc ggc aag tgt gtg cca gca tcg aat gtg act      288
Thr Phe Lys Glu Glu Gly Gly Lys Cys Val Pro Ala Ser Asn Val Thr
                85                  90                  95

tgt aag gat aac aat ggt ggt tgt gcc cct gaa gct gaa tgt aaa atg      336
Cys Lys Asp Asn Asn Gly Gly Cys Ala Pro Glu Ala Glu Cys Lys Met
            100                 105                 110

acg gac agc aat aaa atc gtc tgt aaa tgt act aaa gaa ggt tct gag      384
Thr Asp Ser Asn Lys Ile Val Cys Lys Cys Thr Lys Glu Gly Ser Glu
        115                 120                 125

cca ctc ttt gag gga gtt ttc tgt agc cac cat cac cat cac cat gga      432
Pro Leu Phe Glu Gly Val Phe Cys Ser His His His His His His Gly
    130                 135                 140

gtt ttc tct agc tcc tcc agc ttc cta agc ttg tcc ttc ttg ttg ctc      480
Val Phe Ser Ser Ser Ser Ser Phe Leu Ser Leu Ser Phe Leu Leu Leu
145                 150                 155                 160

atg ttg ctt ttc ctc ctg tgc atg gag ctt taa taa                      516
Met Leu Leu Phe Leu Leu Cys Met Glu Leu
                165                 170

<210>  20
<211>  170
<212>  PRT
<213>  Artificial

<220>
<223>  Synthetic Construct

<400>  20

Met Lys Ala Leu Leu Phe Leu Phe Ser Phe Ile Phe Phe Val Thr Lys
1               5                   10                  15

Cys Gln Cys Glu Thr Glu Ser Tyr Lys Gln Leu Val Ala Asn Val Asp
            20                  25                  30

Glu Phe Ile Leu Ser Gln Leu Leu Asn Val Gln Thr Gln Leu Leu Thr
        35                  40                  45

Met Ser Ser Glu His Thr Cys Ile Asp Thr Asn Val Pro Asp Asn Ala
    50                  55                  60

Ala Cys Tyr Arg Tyr Leu Asp Gly Thr Glu Glu Trp Arg Cys Leu Leu
65                  70                  75                  80

Thr Phe Lys Glu Glu Gly Gly Lys Cys Val Pro Ala Ser Asn Val Thr
                85                  90                  95

Cys Lys Asp Asn Asn Gly Gly Cys Ala Pro Glu Ala Glu Cys Lys Met
            100                 105                 110

Thr Asp Ser Asn Lys Ile Val Cys Lys Cys Thr Lys Glu Gly Ser Glu
        115                 120                 125

Pro Leu Phe Glu Gly Val Phe Cys Ser His His His His His His Gly
    130                 135                 140

Val Phe Ser Ser Ser Ser Ser Phe Leu Ser Leu Ser Phe Leu Leu Leu
145                 150                 155                 160

Met Leu Leu Phe Leu Leu Cys Met Glu Leu
                165                 170

<210>  21
<211>  127
<212>  DNA
<213>  Artificial

<220>
<223>  127 mer long oligo

<400>  21
gccgtagaag acgtcggtgg tagtggtagt ggtaccgtag aagtcgtcga ggagattgaa      60

gaacccgtag agcaagaaca actaggagta caactagaac atgtcgaagt aaattatttc     120

tagaccc                                                              127


<210>  22
<211>  73
<212>  DNA
<213>  Artificial

<220>
<223>  73 mer forward extension oligo

<400>  22
ccggaattca acacgatcat ctcgaaattg atcgagggca aattccaaga catgttgaac      60

atctcgcagc acc                                                        73


<210>  23
<211>  57
<212>  DNA
<213>  Artificial

<220>
<223>  57 mer reverse oligo

<400>  23
gggagatctt tattaatggt gatggtgatg gtggctgcag aagatgccgt cgaacag        57

SEQUENCE LISTING

<110>  INSITUT PASTEUR
       CENTRE NATIONAL DE LA RECHERCHE SCIENTIFIQUE

<120>  PURIFIED POLYPEPTIDE COMPRISING OR CONSISTING OF A C-TERMINUS
       MSP1 ANTIGEN FROM PLASMODIUM FALCIMPARUM CARRYING A
       GLYCOSYL-PHOSPHATIDYL-INOSITOL GROUP (GPI)

<130>  B6211 - AD/YH/KP

<160>  23

<170>  PatentIn version 3.3

<210>  1
<211>  140
<212>  PRT
<213>  Plasmodium falciparum

<400>  1

Val Thr His Glu Ser Tyr Gln Glu Leu Val Lys Lys Leu Glu Ala Phe
1               5                   10                  15

Glu Asp Ala Val Leu Thr Gly Tyr Glu Phe Asn Thr Ile Ile Ser Lys
            20                  25                  30

Leu Ile Glu Gly Lys Phe Gln Asp Met Leu Asn Ile Ser Gln His Gln
        35                  40                  45

Cys Val Lys Lys Gln Cys Pro Glu Asn Ser Gly Cys Phe Arg His Leu
    50                  55                  60

Asp Glu Arg Glu Glu Cys Lys Cys Leu Leu Asn Tyr Lys Gln Glu Gly
65                  70                  75                  80

Asp Lys Cys Val Glu Asn Pro Asn Pro Thr Cys Asn Glu Asn Asn Gly
                85                  90                  95

Gly Cys Asp Ala Asp Ala Lys Cys Thr Glu Glu Asp Ser Gly Ser Asn
            100                 105                 110

Gly Lys Lys Ile Thr Cys Glu Cys Thr Lys Pro Asp Ser Tyr Pro Leu
        115                 120                 125

Phe Asp Gly Ile Phe Cys Ser Gly Ile Phe Ser Ser
    130                 135                 140

<210>  2
<211>  146
<212>  PRT
<213>  Artificial

<220>
<223>  MSP1-19/His

<400>  2

Val Thr His Glu Ser Tyr Gln Glu Leu Val Lys Lys Leu Glu Ala Phe
1               5                   10                  15

Glu Asp Ala Val Leu Thr Gly Tyr Glu Phe Asn Thr Ile Ile Ser Lys
            20                  25                  30

Leu Ile Glu Gly Lys Phe Gln Asp Met Leu Asn Ile Ser Gln His Gln
        35                  40                  45

Cys Val Lys Lys Gln Cys Pro Glu Asn Ser Gly Cys Phe Arg His Leu
    50                  55                  60

Asp Glu Arg Glu Glu Cys Lys Cys Leu Leu Asn Tyr Lys Gln Glu Gly
65                  70                  75                  80

Asp Lys Cys Val Glu Asn Pro Asn Pro Thr Cys Asn Glu Asn Asn Gly
                85                  90                  95

Gly Cys Asp Ala Asp Ala Lys Cys Thr Glu Glu Asp Ser Gly Ser Asn
            100                 105                 110

Gly Lys Lys Ile Thr Cys Glu Cys Thr Lys Pro Asp Ser Tyr Pro Leu
        115                 120                 125

Phe Asp Gly Ile Phe Cys Ser His His His His His His Gly Ile Phe
    130                 135                 140

Ser Ser
145

<210>  3
<211>  19
<212>  PRT
<213>  Artificial

<220>
<223>  Signal Peptide

<400>  3

Met Lys Ile Ile Phe Phe Leu Cys Ser Phe Leu Phe Phe Ile Ile Asn
1               5                   10                  15

Thr Gln Cys

<210>  4
<211>  21
<212>  PRT
<213>  Artificial

<220>
<223>  Anchor peptide

<400>  4

Ser Ser Asn Phe Leu Gly Ile Ser Phe Leu Leu Ile Leu Met Leu Ile
1               5                   10                  15

Leu Tyr Ser Phe Ile
            20

<210>  5
<211>  564
<212>  DNA
<213>  Artificial

<220>
<223>  PfMSP1p19/His/GPI

<220>
<221>  CDS
<222>  (1)..(564)

<400>  5
atg aag atc ata ttc ttt ttg tgt tcg ttc ctc ttc ttc atc atc aac      48
Met Lys Ile Ile Phe Phe Leu Cys Ser Phe Leu Phe Phe Ile Ile Asn
1               5                   10                  15

acg caa tgt gtg acc cac gag agc tac caa gag ctc gtc aag aaa ctg      96
Thr Gln Cys Val Thr His Glu Ser Tyr Gln Glu Leu Val Lys Lys Leu
            20                  25                  30

gag gcc ttc gag gac gcg gtg ttg acc ggc tac gaa ttc aac acg atc      144
Glu Ala Phe Glu Asp Ala Val Leu Thr Gly Tyr Glu Phe Asn Thr Ile
        35                  40                  45

atc tcg aaa ttg atc gag ggc aaa ttc caa gac atg ttg aac atc tcg      192
Ile Ser Lys Leu Ile Glu Gly Lys Phe Gln Asp Met Leu Asn Ile Ser
    50                  55                  60

cag cac caa tgc gtg aaa aaa caa tgt ccc gag aac tct ggc tgt ttc      240
Gln His Gln Cys Val Lys Lys Gln Cys Pro Glu Asn Ser Gly Cys Phe
65                  70                  75                  80

aga cac ttg gac gag aga gag gag tgt aaa tgt ctg ctg aac tac aaa      288
Arg His Leu Asp Glu Arg Glu Glu Cys Lys Cys Leu Leu Asn Tyr Lys
                85                  90                  95

cag gag ggc gac aag tgc gtg gag aac ccc aac ccg acc tgt aac gag      336
Gln Glu Gly Asp Lys Cys Val Glu Asn Pro Asn Pro Thr Cys Asn Glu
            100                 105                 110

aac aac ggc ggc tgt gac gca gac gcc aaa tgc acc gag gag gac tcg      384
Asn Asn Gly Gly Cys Asp Ala Asp Ala Lys Cys Thr Glu Glu Asp Ser
        115                 120                 125

ggc agc aac ggc aag aaa atc acg tgt gag tgt acc aaa ccc gac tcg      432
Gly Ser Asn Gly Lys Lys Ile Thr Cys Glu Cys Thr Lys Pro Asp Ser
    130                 135                 140

tac ccg ctg ttc gac ggc atc ttc tgc agc cac cat cac cat cac cat      480
Tyr Pro Leu Phe Asp Gly Ile Phe Cys Ser His His His His His His
145                 150                 155                 160

ggc atc ttc agc agc tcc tct aac ttc ttg ggc atc tcg ttc ttg ttg      528
Gly Ile Phe Ser Ser Ser Ser Asn Phe Leu Gly Ile Ser Phe Leu Leu
                165                 170                 175

atc ctc atg ttg atc ttg tac agc ttc att taa taa                      564
Ile Leu Met Leu Ile Leu Tyr Ser Phe Ile

180                 185

<210>  6
<211>  186
<212>  PRT
<213>  Artificial

<220>
<223>  Synthetic Construct

<400>  6

Met Lys Ile Ile Phe Phe Leu Cys Ser Phe Leu Phe Phe Ile Ile Asn
1               5                   10                  15

Thr Gln Cys Val Thr His Glu Ser Tyr Gln Glu Leu Val Lys Lys Leu
            20                  25                  30

Glu Ala Phe Glu Asp Ala Val Leu Thr Gly Tyr Glu Phe Asn Thr Ile
        35                  40                  45

Ile Ser Lys Leu Ile Glu Gly Lys Phe Gln Asp Met Leu Asn Ile Ser
    50                  55                  60

Gln His Gln Cys Val Lys Lys Gln Cys Pro Glu Asn Ser Gly Cys Phe
65                  70                  75                  80

Arg His Leu Asp Glu Arg Glu Glu Cys Lys Cys Leu Leu Asn Tyr Lys
                85                  90                  95

Gln Glu Gly Asp Lys Cys Val Glu Asn Pro Asn Pro Thr Cys Asn Glu
            100                 105                 110

Asn Asn Gly Gly Cys Asp Ala Asp Ala Lys Cys Thr Glu Glu Asp Ser
        115                 120                 125

Gly Ser Asn Gly Lys Lys Ile Thr Cys Glu Cys Thr Lys Pro Asp Ser
    130                 135                 140

Tyr Pro Leu Phe Asp Gly Ile Phe Cys Ser His His His His His His
145                 150                 155                 160

Gly Ile Phe Ser Ser Ser Ser Asn Phe Leu Gly Ile Ser Phe Leu Leu
                165                 170                 175

Ile Leu Met Leu Ile Leu Tyr Ser Phe Ile
            180                 185

<210>  7
<211>  49
<212>  DNA
<213>  Artificial

<220>
<223>  Sig 1

<400>  7
cccggatccg aaaatgaaga tcatattctt tttgtgttcg ttcctcttc                49

<210>  8
<211>  48
<212>  DNA
<213>  Artificial

<220>
<223>  Sig 2

<400>  8
tctcgtgggt cacacattgc gtgttgatga tgaagaagag gaacgaac                 48

<210>  9
<211>  50
<212>  DNA
<213>  Artificial

<220>
<223>  Sig 3

<400>  9
gtgtgaccca cgagagctac caagagctcg tcaagaaact ggaggccttc               50

<210>  10
<211>  50
<212>  DNA
<213>  Artificial

<220>
<223>  Sig 4

<400>  10
cccgaattcg tagccggtca acaccgcgtc ctcgaaaggc ctccagtttc               50

<210>  11
<211>  84
<212>  DNA
<213>  Artificial

<220>
<223>  Bac 1

<400>  11
ggggaattca acatctcgca gcaccaatgc gtgaaaaaac aatgtcccga gaactctggc    60

tgtttcagac acttggacga gaga                                           84

<210>  12
<211>  87
<212>  DNA
<213>  Artificial

<220>
<223>  Bac 2

<400>  12
acaggtcggg ttggggttct ccacgcactt gtcgccctcc tgtttgtagt tcagcagaca    60

tttacactcc tctctctcgt ccaagtg                                        87

<210> 13
<211> 84
<212> DNA
<213> Artificial

<220>
<223> Bac 3

<400> 13
cccaacccga cctgtaacga gaacaacggc ggctgtgacg ccgacgccaa atgcaccgag 60

gaggactcgg gcagcaacgg caag 84


<210> 14
<211> 84
<212> DNA
<213> Artificial

<220>
<223> Bac 4

<400> 14
agaggagctg cagaagatgc cgtcgaacag cgggtacgag tcgggtttgg tacactcaca 60

cgtgattttc ttgccgttgc tgcc 84


<210> 15
<211> 87
<212> DNA
<213> Artificial

<220>
<223> Bac 5

<400> 15
ttctgcagct cctctaactt cttgggcatc tcgttcttgt tgatcctcat gttgatcttg 60

tacagcttca tttaataaag atctccc 87


<210> 16
<211> 23
<212> DNA
<213> Artificial

<220>
<223> Bac 6

<400> 16
gggagatctt tattaaatga agc 23


<210> 17
<211> 22
<212> DNA
<213> Artificial

<220>
<223> Bac 7

<400> 17
ggggaattca acatctcgca gc 22


<210> 18

<211>  45
<212>  DNA
<213>  Artificial

<220>
<223>  Bac 8

<400>  18
gcccaaagat ctttattagc tgcagaagat gccgtcgaac agcgg                    45


<210>  19
<211>  516
<212>  DNA
<213>  Artificial

<220>
<223>  PvMSP1p19/His/GPI


<220>
<221>  CDS
<222>  (1)..(516)

<400>  19
atg aag gcg cta ctc ttt ttg ttc tct ttc att ttt ttc gtt acc aaa      48
Met Lys Ala Leu Leu Phe Leu Phe Ser Phe Ile Phe Phe Val Thr Lys
1               5                   10                  15

tgt caa tgt gaa aca gaa agt tat aag cag ctt gta gcc aac gtg gac      96
Cys Gln Cys Glu Thr Glu Ser Tyr Lys Gln Leu Val Ala Asn Val Asp
            20                  25                  30

gaa ttc ata tta tcc cag ctg cta aat gtg caa act cag tta tta act      144
Glu Phe Ile Leu Ser Gln Leu Leu Asn Val Gln Thr Gln Leu Leu Thr
        35                  40                  45

atg agc tcc gag cac aca tgt ata gac acc aat gtg cct gat aat gca      192
Met Ser Ser Glu His Thr Cys Ile Asp Thr Asn Val Pro Asp Asn Ala
    50                  55                  60

gcc tgc tat agg tac ttg gac gga acg gaa gaa tgg aga tgc ttg tta      240
Ala Cys Tyr Arg Tyr Leu Asp Gly Thr Glu Glu Trp Arg Cys Leu Leu
65                  70                  75                  80

acc ttt aaa gaa gaa ggc ggc aag tgt gtg cca gca tcg aat gtg act      288
Thr Phe Lys Glu Glu Gly Gly Lys Cys Val Pro Ala Ser Asn Val Thr
                85                  90                  95

tgt aag gat aac aat ggt ggt tgt gcc cct gaa gct gaa tgt aaa atg      336
Cys Lys Asp Asn Asn Gly Gly Cys Ala Pro Glu Ala Glu Cys Lys Met
            100                 105                 110

acg gac agc aat aaa atc gtc tgt aaa tgt act aaa gaa ggt tct gag      384
Thr Asp Ser Asn Lys Ile Val Cys Lys Cys Thr Lys Glu Gly Ser Glu
        115                 120                 125

cca ctc ttt gag gga gtt ttc tgt agc cac cat cac cat cac cat gga      432
Pro Leu Phe Glu Gly Val Phe Cys Ser His His His His His His Gly
    130                 135                 140

gtt ttc tct agc tcc tcc agc ttc cta agc ttg tcc ttc ttg ttg ctc      480
Val Phe Ser Ser Ser Ser Ser Phe Leu Ser Leu Ser Phe Leu Leu Leu
145                 150                 155                 160

atg ttg ctt ttc ctc ctg tgc atg gag ctt taa taa                      516
Met Leu Leu Phe Leu Leu Cys Met Glu Leu
                165                 170

<210>  20
<211>  170
<212>  PRT
<213>  Artificial

<220>
<223>  Synthetic Construct

<400>  20

Met Lys Ala Leu Leu Phe Leu Phe Ser Phe Ile Phe Phe Val Thr Lys
1               5                   10                  15

Cys Gln Cys Glu Thr Glu Ser Tyr Lys Gln Leu Val Ala Asn Val Asp
            20                  25                  30

Glu Phe Ile Leu Ser Gln Leu Leu Asn Val Gln Thr Gln Leu Leu Thr
        35                  40                  45

Met Ser Ser Glu His Thr Cys Ile Asp Thr Asn Val Pro Asp Asn Ala
    50                  55                  60

Ala Cys Tyr Arg Tyr Leu Asp Gly Thr Glu Glu Trp Arg Cys Leu Leu
65                  70                  75                  80

Thr Phe Lys Glu Glu Gly Gly Lys Cys Val Pro Ala Ser Asn Val Thr
                85                  90                  95

Cys Lys Asp Asn Asn Gly Gly Cys Ala Pro Glu Ala Glu Cys Lys Met
            100                 105                 110

Thr Asp Ser Asn Lys Ile Val Cys Lys Cys Thr Lys Glu Gly Ser Glu
        115                 120                 125

Pro Leu Phe Glu Gly Val Phe Cys Ser His His His His His His Gly
    130                 135                 140

Val Phe Ser Ser Ser Ser Ser Phe Leu Ser Leu Ser Phe Leu Leu Leu
145                 150                 155                 160

Met Leu Leu Phe Leu Leu Cys Met Glu Leu
                165                 170

<210>  21
<211>  127
<212>  DNA
<213>  Artificial

<220>
<223>  127 mer long oligo

<400>  21
gccgtagaag acgtcggtgg tagtggtagt ggtaccgtag aagtcgtcga ggagattgaa      60

gaacccgtag agcaagaaca actaggagta caactagaac atgtcgaagt aaattatttc     120

tagaccc                                                              127

<210>  22
<211>  73
<212>  DNA
<213>  Artificial

<220>
<223>  73 mer forward extension oligo

<400>  22
ccggaattca acacgatcat ctcgaaattg atcgagggca aattccaaga catgttgaac      60

atctcgcagc acc                                                         73

<210>  23
<211>  57
<212>  DNA
<213>  Artificial

<220>
<223>  57 mer reverse oligo

<400>  23
gggagatctt tattaatggt gatggtgatg gtggctgcag aagatgccgt cgaacag         57

## Description

The present invention relates to a purified polypeptide comprising or consisting of a C-terminus MSP1 antigen from *Plasmodium falciparum* carrying a Glycosyl-phosphatidyl-inositol group (GPI), an immunogenic composition and a vaccine against a *Plasmodium* infection comprising said purified polypeptide.

The present invention also relates to a nucleic acid molecule which encodes the amino acid sequence of said purified polypeptide, an expression vector and an expression system for the expression of said purified polypeptide.

Malaria is the world's most important parasitic infectious disease, in terms of the number of exposed and infected individuals. It causes an estimated 2-3 million deaths per year, primarily among children under 5 years old in Africa, and constitutes a heavy economic burden in affected communities. Among the four species of *Plasmodium* responsible for human malaria, *Plasmodium falciparum* causes by far the most morbidity and mortality. The rapidly-increasing incidence of drug-resistant parasite strains, as well as insecticide-resistant mosquito vectors, have drastically increased the urgency of producing an effective vaccine to combat this wide spread plague of poverty. The asexual blood stage of the *Plasmodium* life cycle is responsible for the clinical symptoms and pathology of malaria. The extra-cellular merozoite is the infectious form of the parasite for red blood cells, and its surface proteins are obvious vaccine candidates, since many are likely to play essential roles in the erythrocyte invasion process, and they are accessible targets for humoral immune system effectors (antibodies and complement).

Among the most promising blood stage vaccine candidates is the major merozoite surface protein 1 (MSP1), which has been studied most extensively in *Plasmodium falciparum,* although homologues exist for all other *Plasmodium* species studied, such as for *Plasmodium vivax* for example. It is synthesized during schizogony as a 185-215 kDa protein (depending on the species), which is attached to the merozoite plasma membrane by a C-terminal glycosyl-phosphatidyl-inositol (GPI) moiety [1]. During merozoite maturation, MSP1 undergoes two successive proteolytic cleavages, giving rise to an 11 kDa C-terminal peptide, known as MSP1 p19 due to its migration on SDS-PAGE. MSP1p19 with its GPI anchor is the only part of MSP1 remaining on the merozoite in newly invaded erythrocytes [2] reviewed in [3]. The precise function(s) of MSP1 and/or MSP1p19, although unknown, appear to be essential for erythrocyte invasion and parasite survival, since no viable parasites can be recovered following MSP1 knock-out, although surprisingly, PfMSP1p19 can be complemented by its *P. chabaudi* homologue [4]. Unlike many polymorphic *Plasmodium* merozoite surface proteins [5], PfMSP1p19 is highly conserved in parasites from diverse geographical locations [6]; [7]; [8]; [9], a feature with obvious advantages for a vaccine candidate. MSP1p19 in all species is highly cysteine rich and is composed essentially of two tandem, closely associated epidermal growth factor-like domains. Although the amino acid sequence of MSP1p19 varies considerably among different *Plasmodium* species, its 3-dimentional structure appears to be remarkably conserved [10]; [11]. Indeed its function appears to depend exclusively on its structure than to any given amino acid sequence [4].

In studies carried out in endemic regions, PfMSP1 p19 specific antibodies in the sera of exposed individuals have been associated with protection against high levels of parasitemia and clinical episodes [12]; [13]; [14]; [15]; [16]; [17]. In addition, anti-MSP1 antibodies in human immune sera have been shown to inhibit parasite growth *in vitro* [18]. More importantly, using transfected *P. falciparum* and *P. chabaudi* parasite lines with reciprocal allelic replacements of their divergent MSP1p19 gene sequences (43% homology), O'Donnell et al. [19] could show that antibodies specific for this domain in *P. falciparum* immune human sera or *P. chabaudi* immune mice, represented a major component of the growth inhibition, or invasion inhibitory responses in parasite cultures.

Several vaccination trials have been carried out in primate models using varying recombinant versions of C-terminal MSP1 (p42 and/or p19) in association with a number of different adjuvants, which have provided some indication of parameters that may be important for protective efficacy. None of the C-terminal PfMSP1 recombinant proteins expressed in *E. coli* have shown evidence of protective efficacy in primates [20]; [21]; [22]). Protective efficacy conferred by yeast recombinant versions of PfMSP1 p19 has been highly inconsistent, depending on the subspecies of *Aotus* used [20], the challenge dose of infected red blood cells [23], and the exact nature of the recombinant construct [22]; [24]. (In contrast, recombinant C-terminal MSP1 proteins produced in the baculovirus expression system have consistently shown good protective efficacy in primates [25]; [26]; [27].)

Many parasitic protozoa with extra-cellular phases in their life cycles, such as *Plasmodium,* appear to express a majority of their surface proteins such that a C-terminal hydrophobic signalling sequence has been replaced with GPI moieties [28]. The primary function of GPI structures is to mediate a stable association of surface proteins to the plasma membrane, such that the functional ectoplasmic domain faces the external environment, in relative isolation with intracellular components. In particular, several *P. falciparum* merozoite surface proteins have GPI anchors, including MSP1, MSP2, MSP4 and MSP5 [28], the structure of which has been determined for MSP1 and MSP2 [1]. Recently *P. falciparum* GPIs have been implicated in the pathology of clinical malaria disease by inducing TNFα secretion, leading to fever and the up-regulation of capillary epithelial adhesion molecules that can bind infected erythrocytes causing capillary obstruction and associated pathologies [29]; [30]; [31]; [32, 33]; [34]. Indeed, individuals who live in malaria endemic areas can develop a specific anti-*P. falciparum* GPI antibody response, which is correlated with age and, in some cases, protection against disease [34]; [35]; [36]. In addition, mice immunized with synthetic *P. falciparum* GPI showed enhanced survival and reduced *P. berghei* induced symptoms of malaria. Moreover, antibody directed against this synthetic GPI could neutralize *in vitro* pro-inflammatory activity by *P. falciparum* [37].

In humans, immune responses to MSP1p19 as well as to *P. falciparum* GPIs have been associated with protection against clinical malaria [12]; [13]; [34]. The inventors were interested in developing means that can be encompassed in a vaccine candidate, which could permit to elicit an anti-toxic as well as an antiparasitic immunity, which could have an improved efficiency with respect to the immune response observed in various models, against soluble baculovirus MSP1 p19.

Martinez et al [53] expressed a "recombinant ookinete surface antigen Pbs21 of *Plasmodium berghei* when prepared in a baculovirus SP21 insect cell expression system». The Pbs21 antigen expressed in the baculovirus system was disclosed by Martinez et al, to be produced in the cytoplasm of Sf21 cells, on their surface and in the extracellular medium. The expressed antigen was affinity purified starting from Sf21 cells resuspended in a solubilization buffer. Immunogenicity of the purified recombinant antigen was tested in mice and compared with immunogenicity of the native Pbs21 protein. The recombinant antigen was found to be less immunogenic than the native Pbs21. When discussing the obtained results, Martinez et al reported that recombinant Pbs21 (1-213) bearing the *Spodoptera* GPI anchor was less immunogenic but that the data obtained would suggest that the conformation and the presence of a GPI anchor is critical to the immunogenicity. It is furthermore disclosed that the majority of the response induced is to the folded polypeptide and not its post-translational additions.

However, although, several GPI-anchored proteins have already been expressed in an expression system, the capacity of expression systems to reproduce this anchor for the *Plasmodium* merozoite surface protein MSP1 remains controversial.

The inventors have been able to express a polypeptide from the MSP1 antigen of *Plasmodium,* especially of *P. falciparum,* in eucaryotic cells, and have been able to purify it in conditions enabling the GPI anchor brought by the expression cells to be retained on the polypeptide, hence allowing its use in immunogenic assays.

The present invention relates to a purified polypeptide comprising or consisting of a C-terminus MSP1 antigen from *Plasmodium* carrying a Glycosyl-phosphatidyl-inositol group (GPI).

The term "C-terminus MSP1 antigen" is used to describe a portion of the known MSP1 protein of *Plasmodium,* which is at the carboxy-terminus of said protein and has less than 200 amino acids, preferably less than 150 amino acids. In a particular embodiment of the present invention, said purified polypeptide from the MSP1 antigen is MSP1-19 peptide from *Plasmodium.* The MSP1-19 polypeptide (also designated as peptide) has been described in a specific example; the C-terminus MSP1 antigen from *Plasmodium* carries (or anchors) a Glycosyl-phosphatidyl-inositol group (GPI) which is linked by covalent bonding.

When, the purified polypeptide comprises said C-terminus MSP1 antigen, it is nevertheless a portion of the native MSP1 antigen, i.e. it has an amino acid sequence which is a fragment of the complete sequence of the MSP1 antigen.

In a particular embodiment of the present invention, the "C-terminus MSP1 antigen" is a fragment of the MSP1 C-terminal polypeptide, comprising or consisting from 10 to 200 amino acids and preferably from 10 to 150 amino acids.

In another particular embodiment of the present invention, the MSP1 antigen is a *Plasmodium falciparum* antigen or a *Plasmodium vivax* antigen.

Optionally, this C-terminus MSP1 antigen is recombined with a different peptide or polypeptide, e.g. a peptide or polypeptide originating from another antigen.

In a particular embodiment of the present invention, the purified polypeptide is a recombinant polypeptide expressed in a eukaryotic cell where it is anchored in the plasma membrane through a GPI group synthesized by said eucaryotic cell. The polypeptide of the invention carries the GPI group.

In yet a particular embodiment of the present invention, MSP1-19 peptide has the following amino-acid sequence:

VTHESYQELVKKLEAFEDAVLTGYEFNTIISKLIEGKFQDMLNISQHQCVKK
QCPENSGCFRHLDEREECKCLLNYKQEGDKCVENPNPTCNENNGGCDA
DAKCTEEDSGSNGKKITCECTKPDSYPLFDGIFCSGIFSS (SEQ ID NO 1).

In a specific example, the purified polypeptide of the present invention is recognized by conformation-dependent antibodies directed against the MSP1-19 peptide. Conformation-dependent antibodies have been described in [11] and can be obtained after intraperitoneal immunization of BALB/c mice with Sf9 cells infected with a recombinant baculovirus coding for PfMSP1 p19, or PvMSP1 p19.
In a specific example, the MSP1-19 peptide reproduces the disulphide bonded double EGF-like domain structure of native MSP1p19. Said EGF-like domain, when folded correctly, possesses six disulphite bridges and appears extremely stable and highly resistant to proteolysis. Although this domain is shown to present considerable sequence variation in different Plasmodium species, its 3-dimensional structure and function nonetheless appear to be remarkably conserved [10]; [11]; [19]. EGF-like domains could particularly be involved in maintaining the characteristic properties of PfMSP1 p19 protein as well as the immunological potency thereof.

MSP1p19 structural integrity is of critical importance in an immunogenic context because most, if not all, MSP1p19 B-cell epitopes appear to be non-linear and reduction sensitive, since irreversible reduction abolishes antibody recognition.

The purified polypeptide of the present invention can be expressed in insect cells, and purified from the plasma membrane of said cells, in conditions enabling the GPI anchor to be retained on expressed polypeptide.

In a particular embodiment of the invention the polypeptide can be produced in a baculovirus / insect cell expression system.

Insect cells such as *Spodoptera frugiperda* (Sf9) or *Trichoplusia ni* (High Five, H5) insect cells can be used for the expression of the polypeptide.

In a particular embodiment, the polypeptide of the invention has glycosylation groups. Glycosylation is discussed in examples.

The purification of the polypeptide is achieved having recourse to any known technique such as those described in the examples involving the use of detergents e.g. octyl β-glucoside and if required octyl RP-FPLC purification step.

Other eucaryotic cells can be used for the expression of the polypeptide of the invention, including yeast cells, mammalian cells.

In another aspect of the invention, the MSP1-19 peptide of the present invention is expressed with a His-tag and has the following sequence:VTHESYQELVKKLEAFEDAVLTGYEFNTIISKLIEGKFQDMLNIS QHQCVKKQCPENSGCFRHLDEREECKCLLNYKQEGDKCVENPNPTCNE NNGGCDADAKCTEEDSGSNGKKITCECTKPDSYPLFDGIFCSHHHHHHGI FSS (SEQ lD NO 2).

A hexa-histidine tag near the C-terminus is useful for purification of the polypeptide using metalloaffinity chromatography. The GIFCS and GIFSS sequences are repeated immediately before and after the hexa-histidine tag to assure the GPI modification signal function.

The invention also relates to a purified variant of the polypeptide defined above, having at least 80% identity with the purified polypeptide of the present invention and modified with respect to said polypeptide as a result of amino acid substitution deletion or addition of a least one amino acid residue, with the proviso that the expressed variant retains the GPI group and the immunogenic properties of said purified polypeptide.

In a specific example, said variant has less than 200 amino acids, preferably less than 50 amino acids.

Such a variant can have the same length as the purified polypeptide of the invention, especially when it comprises substituted amino acid residues only. It can be shorter that the purified polypeptide of the invention, especially when amino acid residues are deleted, for instance it can have up to 10 % deleted residues. It can also be longer, especially as a result of added residues, to the extent that it remains shorter than the MSP1 antigen, for example having at most 10 % added residues with respect to the purified polypeptide of the invention.

Substitutions in the purified polypeptide of the invention, giving rise to a variant are preferably conservative substitutions.

The variant polypeptide is especially expressed from a polynucleotide which has a modified nucleotide sequence with respect to the nucleic acid molecule encoding the polypeptide of the invention.

The disclosure which is made in the present application, with respect to the so-called purified polypeptide of the invention, applies in the same way to the variant polypeptide.

Hence, to the exception of the above description of the variant polypeptide with respect to this defined purified polypeptide, when reference is made to the purified polypeptide it also concerns the variant.
The invention also relates to an immunogenic composition comprising the purified polypeptide of the present invention, and a pharmaceutically acceptable vehicule.

The present immunogenic composition can be injected as is, or for convenience of administration, can be added to a pharmaceutically acceptable carrier. Suitable pharmaceutically acceptable carriers will be apparent to those skilled in the art, and include water and other polar substances, including lower molecular weight alkanols, polyalkanols such as ethylene glycol, polyethylene glycol, and propylene glycol as well as non-polar carriers.

The inventors have observed that the GPI group which is present on the purified polypeptide of the invention has an impact on the immunogenicity of the polypeptidic part of the polypeptide, especially provides an adjuvant effect to the immunogenic amino acid sequence.

Accordingly, the purified polypeptide of the invention can be used without adding substances usually added in vaccine compositions to increase the immune response.
In a specific example, the immunogenic composition is thus devoid of additional compounds known as adjuvant of the immune response.

The invention also relates to a vaccine against a *Plasmodium* infection comprising or consisting of an active principle which is the purified polypeptide of the present invention.

The term "vaccine" is used throughout the specification to describe a preparation intended for active immunological prophylaxis, i.e. capable of eliciting an immune response e.g. a cellular and/or humoral response which protects against infection by *Plasmodium falciparum* or its consequences.

The method of administering the vaccine(s) according to the present invention may vary and include intravenous, buccal, oral, transdermal and nasal, among others, but intramuscular or subcutaneous administration is the most common route of administration.
Vaccine compositions of the invention are intended for administration to human.

The present vaccine can be injected as is, or for convenience of administration, can be added to a pharmaceutically acceptable carrier. Suitable pharmaceutically acceptable carriers will be apparent to those skilled in the art, and include water and other polar substances, including lower molecular weight alkanols, polyalkanols such as ethylene glycol, polyethylene glycol, and propylene glycol as well as non-polar carriers.

The active principle which is the purified polypeptide of the present invention should be in an effective amount. The term "effective amount" refers to an amount or concentration of active principle effective to produce a protective immune with respect to the disease malaria. One or several doses may be necessary to achieve the protection sought.

In general, an effective amount of the immunogenic purified polypeptide which is administered to a human patient will vary depending upon a number of factors associated with that patient, including whether the patient previously has been exposed to *Plasmodium falciparum* before. An effective amount of the immunogenic purified polypeptide can be determined by varying the dosage of the product and measuring the resulting cellular and humoral immune and/or therapeutic responses, prior to administration.

In a specific example, said vaccine is devoid of additional compounds known as adjuvant of the immune response.

In a specific example, said vaccine further comprises polymer additives.

One or several further antigens of *Plasmodium* can be added as active principle in said vaccine. Such additional antigens include LSA antigens (including either LSA1, LSA3, LSA5 or combinations thereof), MSP-3, or GLURP.

Said vaccine can be used against infection by *Plasmodium falciparum.*

The invention also relates to a nucleic acid molecule which encodes the amino acid sequence of the purified polypeptide of the present invention.

In a particular embodiment of the invention, the nucleic acid molecule, encodes the amino acid sequence of the purified polypeptide, having the following amino acid sequence: VTHESYQELVKKLEAFEDAVLTGYEFNTIISKLIEGKFQDMLNISQHQCVKK QCPENSGCFRHLDEREECKCLLNYKQEGDKCVENPNPTCNENNGGCDA DAKCTEEDSGSNGKKITCECTKPDSYPLFDGIFCSHHHHHHGIFSS (SEQ ID NO 2). In order to produce the polypeptide of the invention, the amino acid sequence of MSP1-19 expressed in the recombinant cells is transported to the cell membrane and processed to enable GPI binding. Therefore the nucleic acid molecule encoding the polypeptide of the invention comprises a nucleic acid molecule which encodes a signal peptide having the following amino acid sequence: MKIIFFLCSFLFFIINTQC (SEQ lD NO 3), and a nucleic acid molecule which encodes an anchor peptide, that signal for GPI addition having the following amino acid sequence: SSNFLGISFLLILMLILYSFI (SEQ ID NO 4).

According to this aspect of the invention, the nucleic acid molecule of the present invention therefore encodes the following amino acid sequence:

MKIIFFLCSFLFFIINTQCVTHESYQELVKKLEAFEDAVLTGYEFNTIISKLIE
GKFQDMLNISQHQCVKKQCPENSGCFRHLDEREECKCLLNYKQEGDKC
VENPNPTCNENNGGCDADAKCTEEDSGSNGKKITCECTKPDSYPLFDGI
FCSHHHHHHGIFSSSSNFLGISFLLILMLILYSFI (SEQ ID NO 6).

In another embodiment of the invention, the nucleic acid molecule (polynucleotide) of the present invention encodes the amino acid sequence of SEQ ID NO 20.

The sequences which are underligned correspond to putative N-glycosylation sites.

In the present invention, an "anchor peptide" comprises a peptide sequence, for example of about 10 to 35 residues in length which is generally expressed at the carboxy-terminus of the C-terminus MSP1 antigen of the present invention.

The term "signal peptide" is used to describe a 7-30 unit amino acid peptide sequence, preferably about a 15-26 unit amino acid peptide sequence.

The signal peptide is generally expressed at the N-terminus of the C-terminus MSP1 antigen of the present invention.

The nucleic acid molecule of the invention encompasses DNA, either double-stranded or single-stranded DNA depending on the use, cDNA or even RNA.

In a specific example, the nucleic acid molecule of the present invention is optimised for codon usage appropriate in baculovirus expression vector.

The invention also relates to a nucleic acid molecule which can have a nucleotide sequence selected from the sequences presented in the table 1 below:

**Table 1: Primers used for the constructions of recombinant PfMSP1 p19 and PfMSP1 p19+A expressing sequences**

| name | | Sequence |
|---|---|---|
| | | |
| Sig1 | SEQ ID No.7 | CCCGGATCCGAAAATGAAGATCATATTCTTTTTGTGTTCGTT CCTCTTC |
| Sig2 | SEQ ID No.8 | TCTCGTGGGTCACACATTGCGTGTTGATGATGAAGAAGAGG AACGAAC |
| Sig3 | SEQ ID No.9 | GTGTGACCCACGAGAGCTACCAAGAGCTCGTCAAGAAACTG GAGGCCTTC |
| Sig4 | SEQ ID No. 10 | CCCGAATTCGTAGCCGGTCAACACCGCGTCCTCGAAAGGCC TCCAGTTTC |
| Bac1 | SEQ ID No. 1 | GGGGAATTCAACATCTCGCAGCACCAATGCGTGAAAAACA ATGTCCCGAGAACTCTGGCTGTTTCAGACACTTGGACGAGA GA |
| Bac2 | SEQ ID No.12 | ACAGGTCGGGTTGGGGTTCTCCACGCACTTGTCGCCCTCCTG TTTGTAGTTCAGCAGACATTTACACTCCTCTCTCTCGTCCAA GTG |
| Bac3 | SEQ ID No.13 | CCCAACCCGACCTGTAACGAGAACAACGGCGGCTGTGACGC CGACGCCAAATGCACCGAGGAGGACTCGGGCAGCAACGGC AAG |
| Bac4 | SEQ ID No. 14 | AGAGGAGCTGCAGAAGATGCCGTCGAACAGCGGGTACGAGT CGGGTTTGGTACACTCACACGTGATTTTCTTGCCGTTGCTGC C |
| Bac5 | SEQ ID No. 15 | TTCTGCAGCTCCTCTAACTTCTTGGGCATCTCGTTCTTGTTGA TCCTCATGTTGATCTTGTACAGCTTCATTTAATAAAGATCTCC C |
| Bac6 | SEQ ID No. 16 | GGGAGATCTTTATTAAATGAAGC |
| Bac7 | SEQ ID No. 17 | GGGGAATTCAACATCTCGCAGC |
| Bac8 | SEQ ID No. 18 | GCCCAAAGATCTTTATTAGCTGCAGAAGATGCCGTCGAACA GCGG |

The invention further relates to the polynucleotides corresponding to the amplification products obtained with the primers of Table 1.
The invention also concerns the polypeptides expressed from said amplification products.
The invention also relates to an expression vector for the expression of a purified polypeptide of the present invention comprising one of the nucleic acid molecules described above.

The term "expression vector" is used to describe the means by which nucleic acid, including DNA, cDNA, RNA or variants thereof, more preferably DNA fragments, encoding for a specific peptide or protein, may be introduced into a cell, especially an eucaryotic cell to express or produce the desired protein. Such vectors include any vectors into which a nucleic acid sequence insert encoding for polypeptide of the present invention, is inserted, under the control of sequences necessary for replication of the vector and expression of the insert nucleic acid molecule.

In the present invention, the vector is for example a baculovirus vector, and, in a specific embodiment, a baculovirus vector of the type "BaculoGold viral DNA" commercialized by Pharmingen.

In order to express the desired protein or peptide sequence, the expression vector should contain at least one promoter, at least one operator, at least one terminator codon, and any other sequences which are necessary or useful for the efficient transcription and subsequent translation of the nucleic acid from the vector. These operational elements are well known to those of ordinary skill in the art.

In a specific example, said expression vector is a baculovirus expression vector.

The invention also relates to an expression system for the expression of the purified polypeptide of the present invention comprising said baculovirus expression vector and insect cells.

In a specific example, said cells are *Spodoptera frugiperda* (Sf9) or *Trichoplusia ni* (High Five, Invitrogen) insect cells.

The invention also relates to the recombinant baculovirus PfMSP1 p19/His/GPI deposited on November 10, 2005 under the accession number CNCM 1-3515 at the Collection Nationale de Cultures de Microorganismes (C.N.C.M.) INSTITUT PASTEUR , 25 rue du Docteur Roux, F-75724 PARIS CEDEX 15, and containing the nucleic acid sequence encoding the polypeptide of SEQ ID NO 6. The nucleic acid sequence encoding the polypeptide of the recombinant baculovirus PfMSP1p19/His/GPI was constructed from the nucleotide sequence encoding the native MSP1p19 protein of *Plasmodium falciparum.*

The invention also relates to the recombinant baculovirus PvMSP1p19/His/GPl deposited on November 10, 2005 under the accession number CNCM l-3516 at the Collection Nationale de Cultures de Microorganismes (C.N.C.M.) INSTITUT PASTEUR , 25 rue du Docteur Roux, F-75724 PARIS CEDEX 15, and containing the nucleic acid sequence encoding the polypeptide of SEQ ID NO 20. The nucleic acid sequence encoding the polypeptide of the recombinant baculovirus PvMSP1 p19/His/GPI was constructed from the nucleotide sequence encoding the native MSP1p19 protein of *Plasmodium vivax.*

### Brief description of the drawings

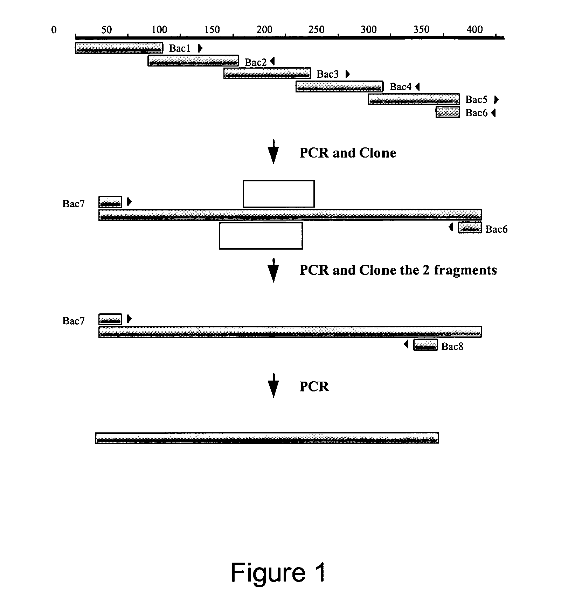

Fig. 1. Strategy used for recoding the C-terminal PfMSP1 sequence in a synthetic gene for the constructions used for the expression of the secreted and the anchored form of the protein MSP1-19.

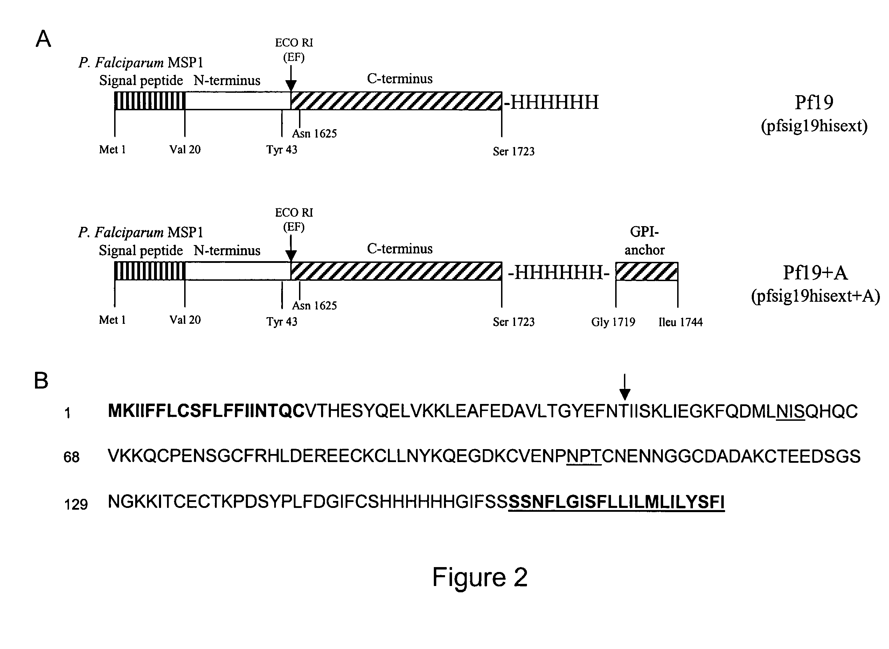

Fig. 2. Structure of the baculovirus constructs expressed in insect cells. A) Pf19: construction used for the expression of the secreted form of the peptide MSP1-19. Pf19+A: construction used for the expression of the anchored form of the protein MSP1-19. Amino acids were numbered according to Chang *et al.,* 1988 [39]. B) Amino acids sequence of SEQ ID NO 6 used to express pf19+A. The peptide signal is in bold, the sequence signal coding for the GPI attachment is in bold and underlined, glycosilation sites are underlined and the arrow indicates the begining of the C-terminal part sequence of MSP1-19.

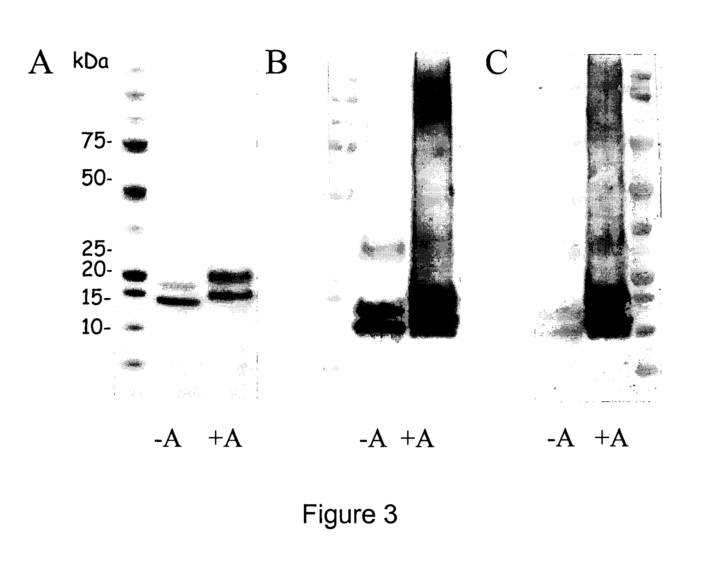

Fig 3. Analysis of purified anchored pf19 +A (+A) and secreted pf19 (-A) forms of MSP1-p19 recombinant proteins by A) coomassie blue staining in reduced conditions; B) immunobloting with a monoclonal antibody G17-12 in non-reduced conditions and C) immunobloting with a hyperimmune sera in non-reduced conditions. Molecular weights are given in kDa.

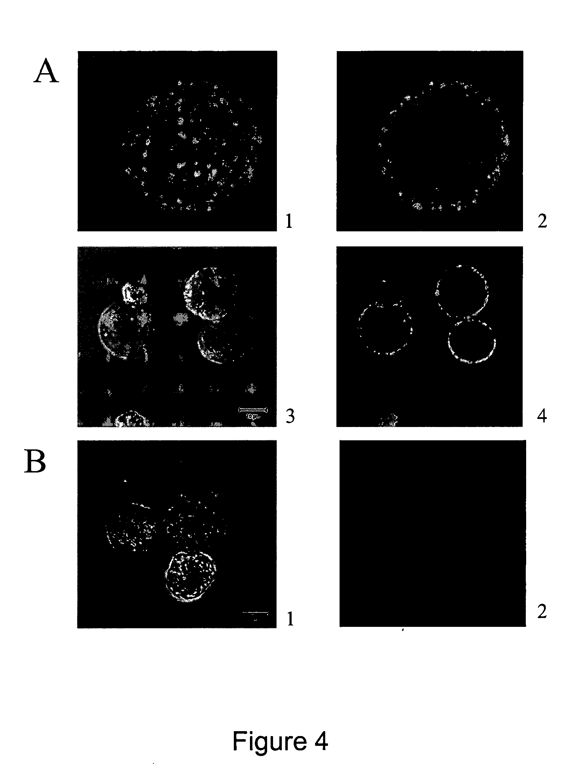

Fig 4. Confocal micrographs of Pf 19+A (A) and pf19 (B) localization in HF (A.1,A.2) or Sf9 (A.3, A.4, B.1, B.2) non-fixed cells infected with recombinant baculovirus for 36 h. A.1 represents a three-dimentional composite of florescence from a whole cell, when A.2 and A.4 represents the central sections of the cells. Similar localization at the surface of the cells was observed in sf9 cells or HF cells for pf19+A when no fluorescent signal was recovered for pf19, either for Sf9 or HF cells.

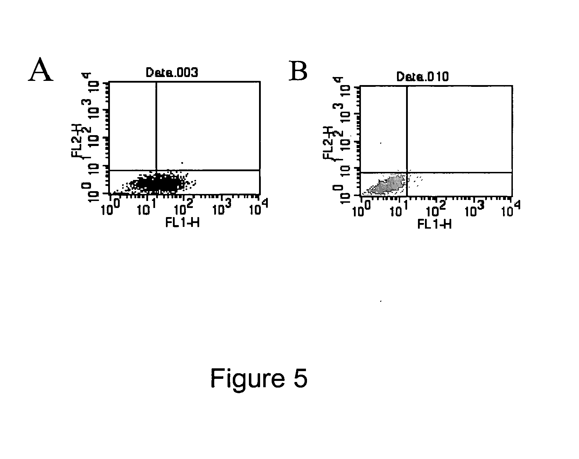

Fig 5. Flow cytometry analysis of the surface expression of pf19+A (A) and Pf19 (B) on live sf9 cells infected for 48h with recombinant baculovirus pfsig19hisext+A and pfsig19hisext, respectively, and first gated for low propidium iodide staining. The Same result was recovered for HF cells.

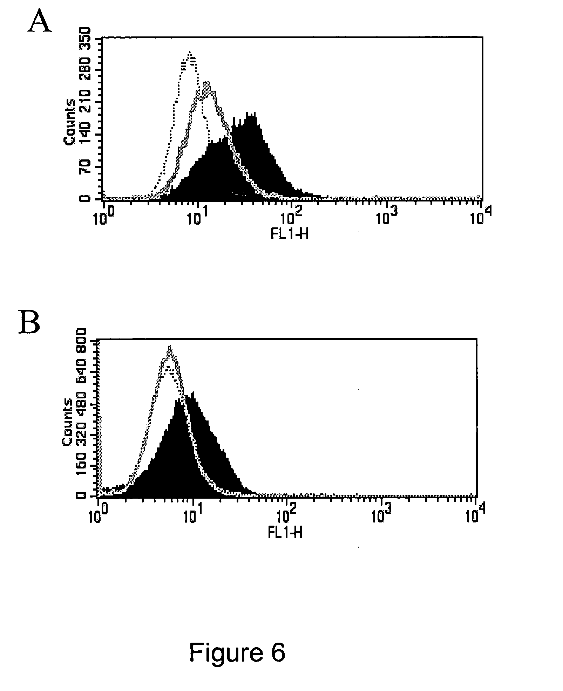

Fig 6. Flow cytometry analysis of surface level of MSP1-p19 on sf9 cells (A) and HF cells (B) infected with recombinant baculovirus pfsig19hisext+A for 48h. Expression of Pf19+A was evaluated with the monoclonal antibodies G17-12 with (thin curve) and without (filled area) treatment by PIPLC. The broken curve represents negative control with a primary irrelevant antibody.

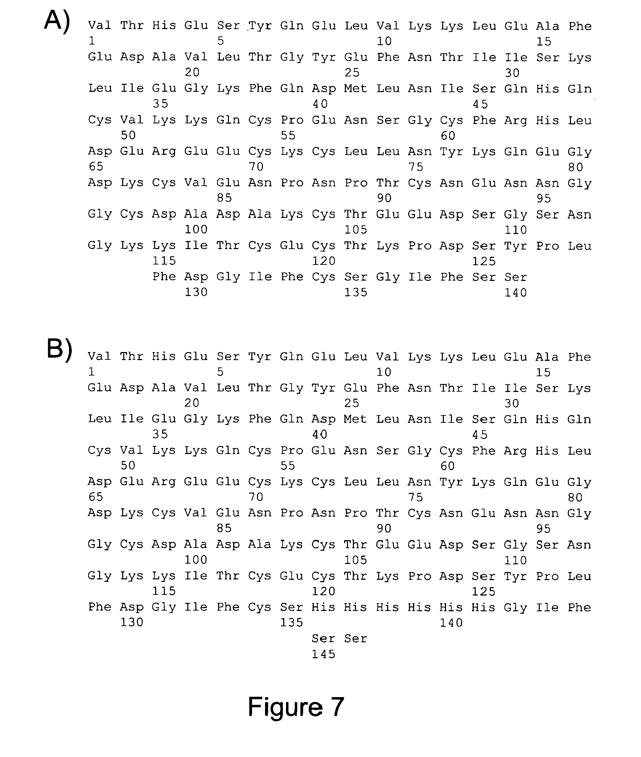

Fig 7 : Fig7A) is the amino acid sequence of the peptide MSP1-19 of SEQ ID NO 1originating from *Plasmodium falciparum.* Numbering of the amino acids is indicated under amino acid residues.
Fig7B) is the amino acid sequence of the peptide MSP1-19 of SEQ ID NO 2 originating from *Plasmodium falciparum* wherein an Histidine Tag has been inserted. Numbering of the amino acids is indicated under amino acid residues.

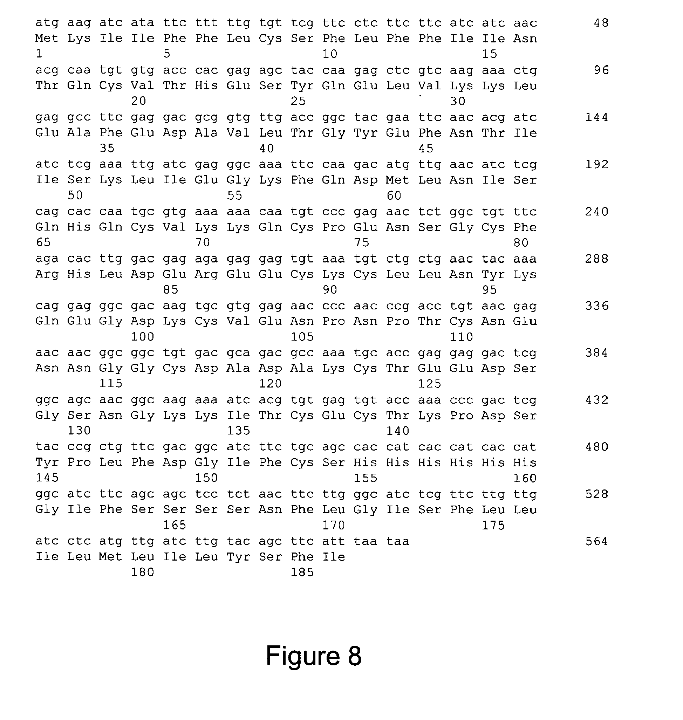

Fig 8 displays the nucleic acid sequence (of SEQ ID NO 5) encoding the polypeptide of SEQ ID NO 6, which constitutes the insert of the Baculovirus PfMSP1 p19/His/GPI deposited at the CNCM on November 10, 2005 under the accession number I-3515. Said polypeptide originates from the *Plasmodium falciparum* MSP1p19 protein, that was modified by the insertion of an Histidine Tag, by the addition of a signal peptide of SE ID NO 3 at its N-terminal end, and by the addition of an anchor peptide of SEQ ID NO 4 at its C-terminal end. The first line indicates the nucleotide sequence, grouped by codons; the second line indicates the amino acid sequence corresponding to the above codons with the three-letter code. Numbering of the nucleic acids is at the right end of the first line, whereas numbering of the amino acids is indicated under amino acid residue (third line).

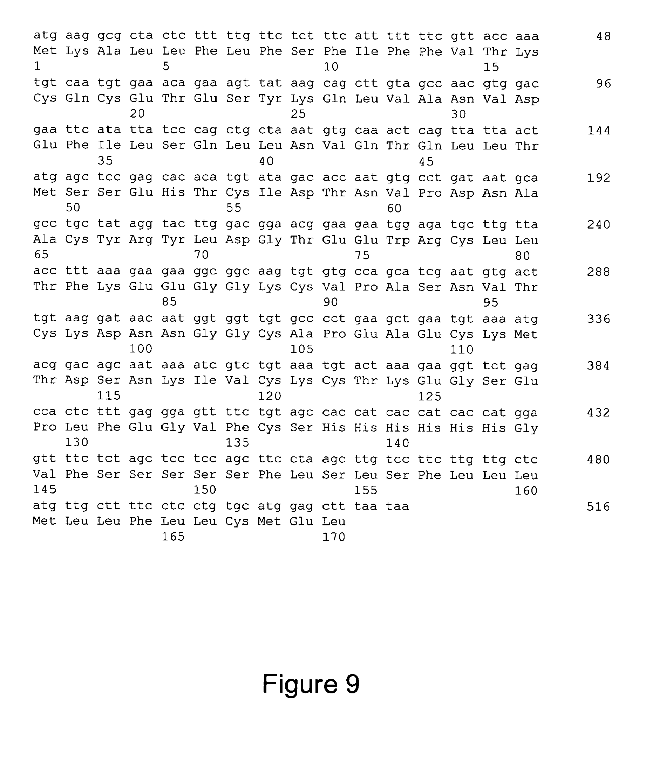

Fig 9 displays the nucleic acid sequence (of SEQ ID NO 19) encoding the polypeptide of SEQ ID NO 20, which constitutes the insert of the Baculovirus PvMSP1 p19/His/GPI deposited at the CNCM on November 10, 2005 under the accession number I-3516. Said polypeptide originates from the *Plasmodium vivax* MSP1 p19 protein, that was modified by the insertion of an Histidine Tag, by the addition of a signal peptide of SE ID NO 3 at its N-terminal end, and by the addition of an anchor peptide of SEQ ID NO 4 at its C-terminal end. The first line indicates the nucleotide sequence, grouped by codons; the second line indicates the amino acid sequence corresponding to the above codons with the three-letter code. Numbering of the nucleic acids is at the right end of the first line, whereas numbering of the amino acids is indicated under amino acid residue (third line).

### Examples

### Example 1: Characterization of secreted and GPI-modified baculovirus recombinant Plasmodium falciparum C-terminal Merozoite Surface Protein 1 (PfMSP1p19), a leading malaria vaccine candidate

Here we have focused on the baculovirus / insect cell expression system for the production of two recombinant analogs of PfMSP1p19 to use in preclinical validation studies that would justify undertaking human clinical trials for this promising malaria vaccine candidate. In this context the baculovirus expression system has two major advantages. First, and most importantly, it appears to reproduce accurately and quantitatively the highly complex disulphide bonded double EGF domain structure of native MSP1p19.Indeed, the crystal structures of both *P. cynomolgi* and *P. falciparum* MSP1p19 were solved using soluble baculovirus produced proteins lacking the extreme C-terminal hydrophobic amino acid sequence, and consequently were secreted into the culture supernatants [38]; [10]; [11]. MSP1p19 structural integrity is of critical importance in a vaccine context because most, if not all, MSP1p19 B-cell epitopes appear to be non-linear and reduction sensitive, since irreversible reduction abolishes antibody recognition. Secondly, we show unambiguously here that in Sf9 or Hi5 insect cells infected with recombinant baculovirus containing the entire C-terminal PfMSP1 coding sequence, PfMSP1p19 is found exclusively on the cell surface or in an intracellular location, rather than secreted into cell culture supernatants. PfMSP1p19 expressed on the cell surface can be at least partially solubilized by phospho-inositol phosopho-lipase C (PIPLC), which specifically cleaves GPI structures. PfMSP1p19-GPI has been purified and characterized prior to use in comparative immunogenicity studies (Example 2). The soluble baculovirus PfMSP1p19 described here is currently undergoing cGMP process development for use in human clinical trials.

### 1-1-Materials and methods

### Insect cell and baculovirus culture

*Spodoptera frugiperda* (Sf9) and *Trichoplusia ni* (High Five, Invitrogen) insect cells were grown in monolayer or suspension cultures at 27°C, respectively in SF-900 serum-free medium (Gibco-BRL) or Insect-XPress protein-free medium (BioWhittaker) supplemented with 4mM glutamine (Gibco-BRL). High titer viral stocks (10⁸ pfu/ml) were produced in Sf9 cells cultured in SF-900 medium supplemented with 5% foetal calf serum (Eurobio; decomplemented 30 min at 56°C) and stored at 4°C.

### Construction of recombinant baculovirus

To optimize PfMSP1p19 expression in the baculovirus system, analogs of the *P. falciparum* genome sequences used (Uganda-Palo-alto isolate, [39]) for the constructions described below (28% G-C), were synthesized with a codon usage corresponding more closely to that of the baculovirus polyhedrin gene (57% G-C), which is replaced by recombinant sequences. Table 2 shows a comparison of parasite genome codons and those used in the synthetic sequences derived from PfMSP1 (50% G-C).

First, a recodoned fragment corresponding to the PfMSP1 signal sequence (19 residues) as well as the 24 conserved N-terminal amino acid residues of processed PfMSP1 (VTH ----- TGY) was synthesized using 4 overlapping oligos:
Pfsig1 of SEQ ID NO 7 (49mer) = CCCGGATCCGAAAATGAAGATCATATTCTTTTTGTGTTCGTTCCTCTTC;
Pfsig2 of SEQ ID NO 8 (48mer) = TCTCGTGGGTCACACATTGCGTGTTGATGATGAAGAAGAGGAACGAAC;
Pfsig3 of SEQ ID NO 9 (50mer) = GTGTGACCCACGAGAGCTACCAAGAGCTCGTCAAGAAACTGGAGGCCTTC;
Pfsig4 of SEQ ID NO 10 (49mer) = CCCGAATTCGTAGCCGGTCAACACCGCGTCCTCGAAAGGCCTCCAGTTTC.

Constructions were assembled as previously described [38] based on restriction sites included in the oligos used to construct the synthetic gene, such that the sequence coding for PfMSP1 signal and the 43 N-terminal amino acids of MSP1 of *Plasmodium Falciparum* were inserted into the Bam HI-Eco RI site on the pVL1393 transfer vector (Invitrogen), creating Pfsig-pVL1393. The 43 N-terminal amino acids inserted in Pfsig-pVL1393 correspond to the fragment of MSP1 ranging from Met₁ to Tyr₄₃, with a substitution of Leu to Phe in position 35. Additionally, the 43 N-terminal amino acids are followed by GluPhe, due to the EcoRI site that constitutes the bond with a sequence coding for a MSP1 fragment of *P. falciparum* between Asn₁₅₉₇ and Ser₁₇₀₅.

In a second step, the synthetic gene corresponding to the PfMSP1p19 naturally processed C-terminal fragment (NISQH on N-terminus) was constructed using a combination of long overlapping oligonucleotides and PCR as shown in Figure 1. The sequences of the oligo nucleotides were as follows:
Bac1 of SEQ lD NO 11 (84mer)= 5'GGGGAATTCAACATCTCGCAGCACCAATGCGTGAAAAAACAATGTCC CGAGAACTCTGGCTGTTTCAGACACTTGGACGAGAGA 3';
Bac2 of SEQ ID NO 12 (87mer) = 5'ACAGGTCGGGTTGGGGTTCTCCACGCACTTGTCGCCCTCCTGTTTG TAGTTCAGCAGACATTTACACTCCTCTCTCTCGTCCAAGTG 3' ;
Bac3 of SEQ ID NO 13 (84mer) = 5'CCCAACCCGACCTGTAACGAGAACAACGGCGGCTGTGACGCCGAC GCCAAATGCACCGAGGAGGACTCGGGCAGCAACGGCAAG 3' ;
Bac4 of SEQ ID NO 14 (84mer) = 5'AGAGGAGCTGCAGAAGATGCCGTCGAACAGCGGGTACGAGTCGGG TTTGGTACACTCACACGTGATTTTCTTGCCGTTGCTGCC 3' ;
Bac5 of SEQ ID NO 15 (87mer) = 5'TTCTGCAGCTCCTCTAACTTCTTGGGCATCTCGTTCTTGTTGATCCTC ATGTTGATCTTGTACAGCTTCATTTAATAAAGATCTCCC 3' ;
Bac6 of SEQ ID NO 16 (23mer) = 5'GGGAGATCTTTATTAAATGAAGC 3' ;
Bac7 of SEQ ID NO 17 (22mer) = 5' GGGGAATTCAACATCTCGCAGC 3';
Bac8 of SEQ ID NO 18 (45mer) = GCCCAAAGATCTTTATTAGCTGCAGAAGATGCCGTCGAACAGCGG.

The product obtained using Bac6 and Bac7 for PCR corresponded to the complete C-terminal MSP1 sequence including the 21 hydrophobic amino acids that signal for GPI addition followed by 2 consecutive TAA stop codons. A construction designed to carry both a hexa-histidine tag near the C-terminus for purification using metalloaffinity chromatography, and the PfMSP1 hydrophobic sequence signaling for GPI modification, was then produced using a 127mer long oligo (GCCGTAGAAGACGTCGGTGGTAGTGGTAGTGGTACCGTAGAAG TCGTCGAGGAGATTGAAGAACCCGTAGAGCAAGAACAACTAGGAGTA CAACTAGAACATGTCGAAGTAAATTATTTCTAGACCC) of SEQ ID NO 21 and PCR to place the recodoned hydrophobic PfMSP1 C-terminal sequence after the hexa-histidine codons (Figure 2). The GIFCS and GIFSS codons were repeated immediately before and after the hexa-his tag to assure the GPI modification signal function, while retaining the cys necessary for EGF domain structure in the former, and changing cys to ser in the latter to avoid undesirable S-S bonding (Figure 2). This construct included, in addition, an extension of 16 amino acid residues upstream from the NISQH processed p19 fragment (Palo Alto allele = NTIISKLIEGKFQDML), which was made using a « forward extension » oligo (73mer) of SEQ ID NO 22: 5'CCGGAATTCAACACGATCATCTCGAAATTGATCGAGGGCAAATTCCA AGACATGTTGAACATCTCGCAGCACC 3'. After amplification, the resulting DNA fragment was then cloned into the *Eco*RI/*Bg*/II) site of Pfsig-pVL1393 containing the signal peptide of *P. falciparum,* creating GPI-PfMSP1p19-pVL1393 coding for the anchored form of MSP1-p19. The product obtained using Bac7 and Bac8 corresponded to the C-terminus of the soluble secreted form of PfMSP1p19, lacking the C-terminal 21 hydrophobic residues, also with 2 TAA stop codons. PfMSP1 p19 constructs designed to be secreted with a C-terminal hexa-histidine tag were produced by PCR from the synthetic gene using a « reverse » oligo encoding the soluble FCS PfMSP1 p19 C-terminus followed by 6 histidine codons and the double TAA stop codons (57mer) of SEQ lD NO 23: 5' GGGAGATCTTTATTAATGGTGATGGTGATGGTGGCTGCAGAAGATGCC GTCGAACAG 3'. This constructs including also the extension of 16 amino acid residues upstream from the NISQH processed p19 fragment made as described above. The final plasmid, coding for the secreted form of MSP1-p19 and called PfMSP1 p19-pVL1393, was then obtained by cloning this fragment into *Eco*RI/*BGL*I cut Pfsig-pVL1393.

The only non-PfMSP1 sequences in these constructs are the EF residues coded by the EcoRl restriction site joining the N-terminal and C-terminal portions of the MSP1 gene, and the hexa-his purification tag. The both sequences, verified by PCR amplification and sequencing, are then similar except that the one coding for the anchored form pf19+A include the glycosylphosphatidylinositol (GPI) anchor signal sequence at the C terminal end, after the His-tag. Recombinant virus were obtained by gently mixing 15µg of each transfert vector with 50ng of BaculoGold viral DNA (Pharmingen) in 1.5ml of TC100 medium (Gibco) supplemented with 4mM glutamine (Gibco) and 50µg/ml of gentamycine (Gibco) with 50µl of DOTAP (Liposomal Transfection Reagent, Roche) in 1,5ml of the same medium. After a 10 min. incubation step, this mix was then added to 2.5 10⁶ Sf9 cells in T-25 culture flasks and cultured for 6 days with one change of medium after 24h. Recombinant virus was then isolated from the culture supernatant by plaque assay using standard methods [40] and amplified as described above.

The recombinant virus PvMSP1 p19/His/GPI was constructed in accordance with the method described above for PfMSP1 p19/His/GPl construction.
Similary, PvMSP1 p19/His/GPl was constructed from the Baculogold vector (Pharmingen) and a transfer vector pVL1393 (Invitrogen), coding for the first amino acids of MSP1 of *Plasmodium vivax* (Belem isolate [121]) from Met₁ to Asp₃₂. These amino acids are followed by GluPhe, due to the EcoRl site which constitutes bond with a sequence coding for a MSP1 fragment of *P. vivax* between Ile₁₆₀₂ and Ser₁₇₀₄.

The resulting sequence is followed by a sequence encoding six Histidine residues (hexa histidine) and by a sequence coding for the fragment GVFSSSSSFLSLSFLLLMLLFLLCMEL, containing the C.terminus of PvMSP1 carrying the signal sequence for the addition of a GPI moiety. Finally, the above construction is ended by two TAA stop codons.

The construct encoding the secreted form of the protein PfMSP1p19 is available at the Collection Nationale de Cultures de Microorganismes (C.N.C.M.), INSTITUT PASTEUR, 25 rue du Docteur Roux, F-75724 PARIS CEDEX 15, under the accession number I-2041, which was deposited on June 18, 1998.

The construct PfMSP1p19/His/GPI, encoding the *Plasmodium falciparum* protein PfMSP1p19+A (including the anchor GPI), is available at the Collection Nationale de Cultures de Microorganismes (C.N.C.M.), INSTITUT PASTEUR, 25 rue du Docteur Roux, F-75724 PARIS CEDEX 15, and was deposited on November 10, 2005, under the accession number I-3515.

The construct PvMSP1p19/His/GPI, encoding the *Plasmodium vivax* protein PfMSP1p19+A (including the anchor GPI), is available at the Collection Nationale de Cultures de Microorganismes (C.N.C.M.), INSTITUT PASTEUR, 25 rue du Docteur Roux, F-75724 PARIS CEDEX 15, and was deposited on November 10, 2005, under the accession number I-3516.

PfMSP1p19/His/GPI and PvMSP1p19/His/GPI constructs are grown in a SF-900 II culture medium comprising L-Glutamine (GIBCO, Invitrogen Corp.), with 2mM L-Glutamine (GIBCO, Invitrogen Corp.), 50µg/ml Gentamicin (GIBCO, Invitrogen Corp.), 5% Foetal Calf Serum, Sodium Bicarbonate, at pH 6.2, at a temperature of 27-28°C.
Virus suspensions are prepared from monolayers of *Spodoptera frugiperda* (SP9) cells cultures grown in complete medium, with 5% Foetal Calf serum. Cells cultures are inoculated with a small amount of viral suspension, or with a plaque or with the content of a microtiter plate well originating from a limiting dilution cloning procedure.
Cultures are incubated at 27-28°C until the cells lysis i.e., until about 5 to 6 days. Infection is ascertained by the following criteria of evidence: enlarged cells and nuclei, granular appearance and cells lysis after a few days. Culture supernatants are then centrifuged 10 minutes at 4000x g to remove cells fragments.
Finally, titration of the viruses is achieved by the limiting dilution method. The expected titer is about 10⁷ to 10⁸ pfu/ml, and about 6 days are required to allow reading of the results.

### Expression of recombinant proteins

Around 1.6 10⁶ insect cells/ml (either HF or Sf9 cells) were infected with recombinant virus (moi=10) and allowed to grow in monolayer or in suspension cultures, at 27°C for 2 or 3 days for pf19 and pf19+A, respectively. Infections were performed in the same medium as non-infected cells in the presence of 50µg/ml gentamycine.

### Purification of recombinant proteins

*Secreted proteins.* Culture media were centrifuged 15 min. at 3000 rpm to remove cells and cellular debris and dialysed against 20 mM tris-HCl (pH8)/500 mM NaCl before purification on an AKTA purifier system (Amersham Pharmacia Biotech), with passage over a 1 ml Hitrap™ chelating HP column (Amersham Pharmacia Biotech) loaded with CaCl2 0.1 M and equilibrated in the same buffer. The column was extensively washed and bound protein eluted with a linear gradient from 0 to 100% of 20 mM tris-HCL (pH8)/300 mM NaCl containing 200 mM imidazole. Fractions (1ml) were then collected and 13µl samples analysed by electrophoresis and western blotting.
*Anchored protein.* Cells were removed from culture by centrifugation (15 min. at 3000 rpm), washed twice in Phosphate buffered saline (PBS) and frozen at -20°C in the presence of 1X protease inhibitors (Complete EDTA-free, Roche). Membrane-proteins were solubilised in 4 volumes of 60 mM octyl β-glucopyranoside in 20 mM Tris-HCl pH8 in presence of 1 X Complete EDTA-free proteases inhibitors (Roche) for 2-3 hours at room temperature. After a centrifugation step of 45 min. at 15000 rpm, the supernatant was incubated over night with TALON Metal Affinity at 4°C. After an extensive wash of the resin, proteins were eluted with 50 mM Imidazole buffer and the fractions containing the MSP1-p19 protein were precipitated with 1 M ammonium sulphate during one night at 4°C. After a centrifugation step at 3000 rpm for 15 min., the supernatant was submitted to a second purification step, on a Hitrap™ Octyl Sepharose FF column (Amersham Pharmacia Biotech) with an AKTA purifier system (Amersham Pharmacia Biotech). The sample was applied in 20 mM tris HCl, pH8 containing 1 M ammonium sulphate and eluted with a linear gradient from 0 to 100% of 0.05% Triton X-100 and 20 mM Tris Hcl pH8 in 30% propanol-1 performed at a flow rate of 1.5 ml/min.

### N-terminal sequencing analysis and mass spectrometry

Purified proteins were resolved by 4-12 % Nu-Page gels (Invitrogene), transferred to a polyvinylidene difluoride (PVDF) membrane, and sequenced using the Edman degradation method. Soluble protein samples (pf19) were analysed by matrix-assisted laser desorption ionization-time of flight (MALDI-TOF) mass spectrometry using Sinapinic acid matrix. The measurements were carried out on a Voyager-DE STR spectrometer (Applied Biosystems Inc., Framingham, USA) equipped with a nitrogen laser (337 nm).

### Antibodies and immune serum

The monoclonal antibody G17-12, which is specific for a conformational epitope of PfMSP1p19 [11] was obtained after intraperitoneal immunization of BALB/c mice with Sf9 cells infected with a recombinant baculovirus coding for PfMSP1p19. Fluorescein isothiocyanate (FITC)-conjugated goat anti-mouse IgG (Immunotech) and goat anti-mouse and anti-human IgG (H+L) alkaline phosphatase conjugated antibodies (Promega). The irrelevant antibodies used as control in the cytometric assays were mouse anti-SV40 ones purchased by Calbiochem.
The hyperimmune human serum used for immunoblots, from an individual donor living in Libreville (Gabon), was kindly provided by Odile Puijalon.

### Electrophoresis and western blot analysis

Samples were analysed on 4-12% acrylamide Nu-Page gels (Invitrogen), under reducing (with DDT, for Coomassie blue staining) or non-reducing (without DDT, for western blotting) conditions. Protein bands were visualised by Coomassie Blue staining or electrophoretically transferred to nitrocellulose (Schleicher & Schuell). After transfer, blots were blocked with 5% milk powder in Tris-buffered saline (TBS) for 30 min. at room temperature (RT) and incubated for 1 h at RT in 50µg/ml of MAb G17-12 diluted in TBS. Blots were then washed 3 times for 10 min. in 5% milk in TBS, and incubated 1 h at RT with goat anti mouse alkalin phosphatase conjugated antibodies (Promega) diluted 1:7500 in TBS. After washes in TBS, antibody binding was detected with the ProtoBlot NBT/BCIP Color Development System (Promega).
Same procedure was applied for western blots using human hyperimmune serum as the primary antibodies diluted at 1:200 and revealed by goat anti-human alkalin phosphatase conjugated antibodies (Promega) diluted 1:7500 in TBS.

### Confocal microscopy

Infected Sf9 or H5 cells expressing either the anchored or the secreted form of MSP1-p19 were harvested 1, 2 or 3 days post infection. After washing with culture medium and 5% FCS (Foetal Cow Serum) the cells were incubated in 50µg/ml of MAb G17-12 and 0,01 % Na-azide for 45 min. at 4°C. Cells were then washed 2 times and incubated for 45 min. at 4°C in the dark with goat-anti mouse FITC conjugated antibodies diluted 1:7500 with TBS. After three washes, cell pellets were suspended in 15µl of Mowiol and cover-slips mounted on glass slides for an incubation of 3 hours in the dark at 4°C. The confocal analysis of the localisation of MSP1-p19 was then performed with a Zeiss LSM-510 microscope and optical sections were performed at 0.5µm increment. The three-dimensional reconstitutions were carried out using the LSM-51 0 software.

### Flow cytometry

About 5.10⁶ infected HF or Sf9 cells expressing either pf19 or pf19+A were harvested 1, 2 or 3 days after infection and washed in culture medium with 5% FCS. Cells were then incubated in 50µg/ml of MAb G17-12 or anti-SV40 mAb (control) and 0.01% Na-azide for 45 min. at 4°C and washed twice in medium with FCS as above. After an incubation of 45 min. at 4°C in the dark with goat anti-mouse FITC conjugated antibodies diluted 1:7500 with TBS, cells were washed 3 times in medium with FCS, and the pellets were resuspended in 1 ml of PBS with 1µg/ml propidium iodide (Sigma). Surface MSP1-p19 levels of each cell samples were then monitored on a FACScan analyser (Beckton Dickinson, Abingdon, Oxford, U.K.).

### PI-PLC treatment of cells

About 5.10⁶ infected cells expressing the protein pf19+A were washed in PBS and incubated for 1 hour at 37°C with 0.3 unit of PI-PLC (Phosphatidylinositol phospholipase C, Sigma) in 300 µl of PBS. Cells were then washed twice in culture medium with FCS, and stained for flow cytometric analysis as described in the above section. Controls were performed in the same way but without addition of the PI-PLC.

### Inositol and monosaccharide analysis

Prior to the GC-MS analysis, salts and detergents were removed by TCA/cold acetone precipitation. The pellet was resuspended in water and the protein amount was estimated by the BCA protein Assay (Pierce), using Bovine serum albumine as standard. Inositol residue was identified and quantified by GC-MS according to Fergusson *et al.* [41]. Monosaccharide was analysed by GC-MS after methanolysis, re-N-acetylation and trimethylsilylation according to Kamerling *et al.* [42].
GC-MS was performed on a Carlo Erba 8000 Top GC apparatus coupled to a Finnigan Automass II mas spectrometer, using a capillary column (30 mm*0.25 mm) filled with EC-1 (Alltech), gas vector: helium, 2 ml/min., column temperature for Inositol analysis: 40 to 150°C at 15°C/min., 150°C to 200°C at 10°C/min., 200°C to 240°C at 5°C/min. and 240°C for 7 min. Column temperature for monosaccharide analysis: 40 to 100°C at 15°C/min., 100°C to 200°C at 7°C/min., 200°C to 240°C at 15°C/min. and 240°C for 10 min. Electron ionization (E1) spectra were recorded using an ionization energy of 70CV and an ionization current of 0.2 mA.

### 1-2-Results

### Expression and purification of the membrane bound and secreted form of recombinant MSP1-p19 proteins

### Expression and purification

HF and Sf9 cells were infected with the corresponding recombinant virus and MSP1-p19 was produced and purified both as anchored and secreted forms from cell detergent extract and culture medium, respectively. Several detergents were used in order to optimize the solubilization of the recombinant anchored protein and thus the octyl β-glucoside appeared to be the more appropriate one, as previously observed for other proteins anchored to the cell membrane via a GPI moiety, either for insect cells [43] or other cell species [44]; [45]. The purification of the secreted protein form is achieved with the chromatographic metallo-affinity, while, for the cell surface form, an additional octyl RP-FPLC purification step was needed.

The yield of purified protein was evaluated at 3.2 mg/liter of culture medium (around 1.6 10⁹ cells) and 11.2 mg/l for pf19+A and pf19, respectively.

### N-terminal sequencing and mass spectrometry analysis

N-terminal sequence analysis showed that, after purification, only pfMSP1-19 derived sequences are detected, either for Pf19+A or Pf19 preparations. For Pf19+A, results obtained have revealed the presence of at least 2 forms of recombinant MSP1-p19, i.e., 90 % with VTH and 10% with MKI at N-terminal ends (see Fig. 2). The first one is a processing form of the protein which could be located at the cell surface but also in the endoplasmic reticulum, and the second one, which retains the signal peptide sequence, is apparently not processed and remains intracellular. This result is likely due to the fact that protein extraction was done on whole cells and that infected cells may have difficulty in processing an excess of recombinant proteins. When the 2 predominant protein bands (20 and 25KD, see figure 3) were analysed separately, we showed that both of them are processed with 100% of protein containing the sequence VTH at the N-terminal end. Concerning the secreted form of the protein, Pf19, N-terminal sequencing showed that there is only the VTH sequence at the N-terminal end, implicating that all recombinant purified proteins are processed and lost their signal peptide.
MALDI-TOF mass spectrometry performed on the purified Pf19 protein preparations showed one major ion at m/z=16016 and several minor ions including m/z=17054, 17204, 17403 and 17606. The main ion corresponded to non-glycosylated soluble PfMSP1p19 with a VTHES N-terminus. The ion mass increases of 1038, 1184, 1387 and 1590 were in agreement with the detected presence of glycosylated structures, as described below.

### Electrophoresis and western blot analysis

The MAb G17-12 used is specific for PfMSP1-19 as it didn't recognize baculovirus-recombinant PvMSP1-19 of *P. vivax,* while MAb F10-3 specific to PvMSP1-p19 did not react with Pf19+A or Pf19-A (data not shown).
Culture supernatants of the secreted form of Pf19 protein exhibited protein bands in electrophoresis after immunoblotting under non reducing conditions, while no reactive band was observed for culture supernatant of pf19+A infected cells. On the contrary, both cells lysates reacted with MAb G17-12 (data not shown).
As shown in figure 3, the electrophoretic mobility of the purified proteins was lightly greater under non-reducing conditions (fig. 3B, 3C) than in reducing conditions (fig. 3A). This result is expected since the products are very rich in cystein and probably have a more compact conformation under the non-reduced conditions. Under reducing conditions, purified proteins from the Pf19+A infected SF9 or HF cells exhibited two major bands migrating at 20 and 25 KD, at a higher apparent molecular mass than for the secreted form Pf19 (2 protein bands of around 18-22 KD) (fig. 3A). Results obtained after Western immunobloting in non-reduced conditions suggested that all these protein bands correspond to MSP1-p19 and that higher molecular weight bands correspond to MSP1-19 aggregated forms (fig. 3B, 3C). The reactivity with monoclonal antibodies or immune serum was considerably decreased by disulphide reduction, indicating the predominance of non-linear epitopes (not shown). Western blots performed with human hyperimmune serum showed that both proteins (anchored and secreted forms) are recognised by naturally occurring antibodies (fig. 3C). This is strong evidence that the conformation of the recombinant proteins is closely related to that of the native MSP1-19.

### MSP1-p19 recombinant protein can be expressed as a GPI-anchored form at the surface of insect cells.

### Fluorescent microscopy

HF and Sf9 cells expressing the pf19+A form of the protein exhibited clearly surface fluorescence either by confocal microscopy (fig. 4) or IFA (Data not shown) analysis being performed with the monoclonal G17-12 antibody. No surface fluorescent signal was recovered for the cells expressing the pf19 secreted protein form, either in Sf9 or HF cells. As shown in figure 4, the Pf19+A protein seemed to be expressed in a "ponctiform" manner at the surface of the cells, especially in HF cells, while the expression appeared to be more homogenous for the Sf9 cells.

### Flow cytometric analysis

Flow cytometric analysis was carried out on live cells since dead cells absorb non specifically either the antibodies or proteins present in the medium. Thus, cells were then gated for low propidium iodide before analysis. A surface fluorescent signal was detected for cells expressing the Pf19+A protein, whereas no signal was detected for the cells expressing the secreted protein Pf19 (fig. 5).
Recombinant full length C-terminal MSP1, MSP1-p19, produced in insect cells/baculovirus system was designed to be attached to the cell membrane via a GPI anchor. To verify this, the intact infected Sf9 and HF cells were incubated with a PI-Phospholipase C, a phospholipase, which specifically hydrolyses the GPI link and thus releases GPI-anchored protein from the cell surface. Cell surface staining was then checked by flow cytometric analysis using the G17-12 antibody. After 48 h of infection, PI-PLC treatment of the pf19+A expressing cells leads to a decrease of the Sf9 cells surface staining (fig 6A) and to a complete elimination of the surface protein for HF cells (fig 6B). This result confirms the surface localisation of the recombinant protein and demonstrates that at least a part of it is anchored to the insect's cell membrane via a GPI anchor.

### Inositol and carbohydrates analysis

The PfMSP1p19 construct contains two potential N-glycosylation sites, at N162IS and N107PT. Monosaccharide analysis revealed the presence of N-acetyl glucosamine (GlcNAc), mannose (Man) and fucose (Fuc) in the purified soluble PfMSP1 p19 preparations, which are characteristic of insect cell N-glycosylation patterns. However, since N-glycosylation has never been demonstrated to occur with a proline in the second position, it was assumed that in this construct only the NIS site is used, which is consistent with the N-terminal sequencing and mass spectrometry data.
N-glycosylated entities were identified by MALDI-TOF mass spectrometric analyses of lyophilized samples, and resulted in the various ions described above. In particular, minor ions including m/z=17054, 17204, 17403 and 17606 were in agreement with the detected presence of glycan structures containing N-acetylhexosmaine, hexose and deoxyhexose in the following ratios respectively of 2:3:1, 2:3:2, 3:3:2 and 4:3:2. The presence of two additional minor ions at m/z=16222 and 16430 was also consistent with 1 and 2 N-acetylhexosamines added to the core PfMSP1 p19 polypeptide. These could correspond to glycans with a LacNAc unit composed of N-acetylglucosamine and +/- N-acetylglucosamine previously detected in recombinant proteins expressed in *T. ni* cells [43]. Although no galactosylated N-glycans were detected here, the corresponding ion at m/z=16430 is very low intensity, and could be below the detection limit.
Concerning the PfMSP1p19+A construct, quantitative monosaccharide analysis revealed the presence of 3% sugar in the protein preparation, including fucose, mannose and N-acetylglucosamine residues in the respective molar ratio of 0.5:3:1.5. Taking into account the 16kDa molecular mass of the PfMSP1p19+A protein, these data suggested that half of the PsMSP1p19+A preparation is non N-glycosylated and half carries one N-glycan similar to the soluble recombinant form.
The amount of inositol was also quantified by GC-MS analysis, that showed the presence of 0.15 µg of inositol per 100µg of protein. Since the mass of MSP1-19 was estimated at 16kDa, no more than 14% of protein contained one inositol residue, indicating that only a small part of the MSP1-19 membrane bound preparation contains a GPI.

### 1-3-Discussion

### 1) Production and purification

The production and purification procedure of both proteins is relatively rapid, scalable and generate a high amount of proteins. The cells infection conditions described above were found optimal for soluble as well as for membrane bound proteins. Two days of infection were needed in order to obtain high amount of secreted protein, while longer infection time leaded to degradation of the protein. One metallo-affinity chromatography appeared to be sufficient for obtaining a high purity of the protein as proved by the N-terminal sequencing and mass spectrometry analysis. Concerning the membrane bound protein, cytometric analysis have showed that 3 days of infection were required for optimization of the production (Data not shown). This protein was better solubilized under octyl glucoside than Triton X-100 detergent (data not shown). According to Hooper and Turner, this finding suggests the presence of a GPI membrane anchor [46]. Such hypothesis was also supported by Triton X-114 phase partition studies as the pf19+A protein was recovered in the detergent phase, and the secreted form of the protein was located in the aqueous fraction (Data not shown). Such result suggested that the C-terminal alone is responsible for partition into the detergent phase, which is consistent with the presence of a GPI anchor [47]. Following purification by metallo-affinity, the precipitation step eliminated most of the contaminants and octyl sepharose chromatography permitted to isolate two protein forms of MSP1-19 in 2 -3 ml fraction.

At the final step of the purification, N-terminal sequencing showed effectively that there is only MSP1-19 in both preparations and the amount of produced anchored and secreted proteins was 2µg /10⁶ cells and 7µg /10⁶ cells, respectively. Then, baculovirus infected cells are able to produce 3,5-fold more secreted recombinant protein than anchored one. This indicates that processing of the GPI signal peptide and/or attachment of the protein to the cell membrane limited the production of the protein in the baculovirus system. Such a result is not surprising because the GPI biosynthesis pathway is a multi-step process involving many biochemical reactions [48]. In addition, the N-terminal analysis revealed the presence of a 10% proportion of the protein, which retained the signal peptide at the N-terminal end. Moreover, no more than 14 % of the membrane bound form contained a GPI-anchor and we can deduce from such results that around 70 % of the purified protein is retained in the endoplasmic reticulum, without the peptide signal sequence, but with the sequence coding for the GPI attachment. This is not such surprising result as the protein was extracted from whole cells and that some protein is necessarily present in an intra cellular unprocessed or processed (but without the addition of the GPI) forms. The facts that insect cells were boosted in order to produce a lot of protein in a non-physiological manner and that by the late stage of baculovirus infection, some enzymes involved in glycosylation are reduced in activity [49], probably explain the great proportion of such intracellular protein forms. Proteins must be effectively correctly folded before they can exit the endoplasmic reticulum and incorrect folded proteins are retained as it was already described concerning GPI-proteins [50]. However, the final amount of purified protein is higher than previously reported for other anchored proteins expressed in the baculovirus system such as CD59: 0.75-1 µg/10⁶ cells [51] or p97: 0,18µg/10⁶ cells [52].
Immunoblot on cell lysates revealed that a significant proportion of the secreted form of the protein (Pf19) was also retained inside the cells (not shown). This could be due to a deletion in the secretory peptide signal sequence, or inefficient recognition of the signal or to a "staking" effect due to a limited secretory capacity of the cell. However, purification procedure permits to obtain only processed protein and with yields that generally varied from 20-30 mg/L in High Five insect cells.

### 2) Western blot analysis

The western blot analysis performed either with the monoclonal antibodies or the human immune serum revealed that both proteins retained conformational epitopes closely related to those present on the parasite MSP1-p19 protein occurring in natural setting. However, the results also suggested that the recognition by antibodies, either with the monoclonal antibody or the human serum, was better with the anchored form of the protein than with the secreted one. For some authors, this reduced reactivity without the GPI can come from the fact that the addition of the GPI anchor to the polypeptide within the endoplasmic reticulum is important for the conformation of their protein [53]. But we can not exclude a difference between the two proteins due to other post-transductional modifications or due to their presentation to the secondary antibodies on the transfert membrane and the following binding.

### 3) Electrophoresis analysis

Electrophoresis analysis showed a lightly reduced mobility for the anchored one rather than the secreted one, probably due to the presence of the transmembrane part of the protein. The MAb G17 detected the presence of multimeric forms of the purified proteins. Such aggregation form of the protein may be facilitated by the presence of the hydrophobic C-terminal part (either the sequence coding for the GPI and the GPI itself) in the anchored form of MSP1-19.
Secreted protein present mainly 2 major protein bands. No difference in peptide sequence has been observed. MALDI-TOF analysis and carbohydrate detection (Mannose, glucose, Fucose) suggested the presence of an N-glycan probably linked to Asn position (see Fig 1 B). The presence of N-glycans can explain the double band of soluble MSP1-19 with a glycosyled higher molecular weight band and a non-glycosyled lower molecular weight one.
Concerning the anchored form, several hypothesis have been proposed to explain the presence of two protein bands in addition to aggregated protein forms: differences in glycosylations as for pf19, or differences in processing or GPI-anchoring between both molecular species.
Indeed, the PfMSP1p19 products expressed in insect cells appear to be partially mofified post-transcriptionally with typical insect N-glycan entities [64]. These probably account for the difference in SDS-PAGE migration of the different bands seen for soluble PfMSP1p19, which otherwise appear to have identical polypeptide contents. The two "monomer" SDS-PAGE bands for the PfMSP1p19+A products are more difficult to define due to proable variability of N-glycan content, coupled with the presence of two very different C-termini with either a GPI structure or hydrophobic amino acids.
Interestingly, in insect cell glycoproteins, the proximal N-acetylglucosamine residue can be fucosylated at position 6, as in mammalian cells, but also at position 3 (α1,3-fucosylation) which is relatively ubiquitous in plants and insects, but absent in mammalian glycoproteins. This feature renders the corresponding glycoproteins immunogenic in mammals [65], and therefore could be of potential interest for baculovirus recombinant proteins designed to function as human vaccine candidates.

### 4) Baculovirus / insect cells system

It is now well admitted that the baculovirus/ insect cells system represent a system of choice for the expression of recombinant proteins in their native conformation. In addition, there are several examples of GPI-anchored recombinant proteins that can be expressed at insect cells surface in a functionally active form as it as been shown for recombinant GPI-B7-1, a human protein [43] and a GPI-recombinant form of the human protein CD59 [51].
Concerning MSP1, recombinant MSP1 proteins expressed respectively in yeast [20]; [22]; [23]; [67] or in E. coli [20]; [21]; [22] have shown a highly inconsistent or generally poor protective efficacy in primates, with one possible exception [66]. Moreover, ELISA studies conduced with a recombinant PfMSP1p19 protein produced in S. cerevisiae or E. coli [68] have shown that the corresponding PfMSP1 p19 specific antibodies did not correlate with protection against high levels of parasitemia.
Concerning the GPI anchored form of MSP1, a synthetic gene was constructed with a markedly reduced A+T content but, when expressed in mammalian cells or *E*. *coli,* it was not transported to the cell surface [54]. As well, when MSP1 gene was expressed from a recombinant vaccinia virus, the amount of MSP1 found on the surface of the HeLa cells was very low [55]. There is now some clear evidences that membrane-bound proteins are not well transported to the cell surface in transformed mammalian cells [56]. Even when the protein MSP1 was expressed in mammalian cell with a synthetic DNA construct encoding human sequence for N-terminal signal and C-terminal recognition/attachment signal for the GPI-anchor, only a very small amount of the recombinant protein was present on the surface of the cells [57].
However, it will be important, in the future, to evaluate if the presence of N-glycosylations has an impact or not on biological properties of the proteins. Such a finding was effectively mentioned in the mammalian expression system, where the recombinant MSP1 protein presents a high level of N-Glycosylations [57], that is not the case in the native protein and which could potentially interfere with the immunogenicity of the protein.

### 5) GPI group

Recombinant MSP1-19 expressed at the cell surface of infected Sf9 and HF insect cells was cleaved with PI-Phospholipase C (PI-PLC), showed that the protein was correctly attached via a GPI-anchor, as intented in the experimental design. However, no more than 14 % of membrane bound MSP1-19 contains an Inositol residue indicating that the main part of recombinant protein is not transferred to a GPI-anchor and stay in the cell. Such a result can be easily explain by the fact that the infection by the baculovirus kill the insect cells, which become rapidly not able to synthesised the GPI as proposed by Kedees [58]. Theses authors used two constructions for MSP1: one corresponding to the intact C-terminal MSP1 fragment, modified by addition of a signal sequence for secretion, and one similar but in which translation of the GPI-cleavage/aftachment site was abolished by insertion of a stop codon into the DNA. When expressed in insect cells, both proteins could only be detected intracellularly, indicating a lack of secretion as well as transport to the cell surface. As no GPI could be detected, contrary to our results, they concluded that the baculovirus system does not seem to be the system of choice if expression in a native and GPI-anchored form is desired.
Our results show that, especially with Sf9 cells, PI-Phospholipase C do not cleave all the GPI-protein present at the surface of the insect cells. It is possible that these proteins are differentially processed in the two insect cell lines. For example, Hegedus *et al.* showed that Sf9 cell lines are able to express recombinant p97 protein that is GPI-anchored to the cell membrane, while Dipteran cell lines are not able to [59]. Some previous studies realized with insect cells expressing human recombinant protein have already reported that about 60% or 80% of cell surface proteins were cleaved after PIPLC treatment [51]; [43], that can be due to inaccessibility of the anchor [51] or the presence of a PIPLC-resistant form of GPI anchor with some additional acyl groups [60].

It is noteworthy that native MSP1 could not be cleaved from the parasite surface because of the presence of a fatty acid on the inositol ring [1]. Thus, GPI synthesised by the insect cells differs in their structure from those synthesised by the parasite; and it will be essential to evaluate how this recombinant protein can mimic the native one in *in vivo* conditions.

### Example 2 : Immunogenicity studies with GPI-anchored and secreted form of Plasmodium falciparum MSP1-p19, produced in the baculovirus expression system, GPI acting as an adjuvant.

### 2-1-Materials and methods

### Cells, constructions, generation and purification of recombinant proteins

*Trichoplusia ni* (High Five, H5) and *Spodoptera frugiperda* (SF9) insect cells (Invitrogen) grown either in monolayer or in suspension cultures were cultured at 27°C in Insect X-Press medium (BioWhittaker) supplemented with 4mM glutamine (Gibco-BRL) or SF-900 (Gibco-BRL), respectively. The amplification of recombinant virus was done in Sf9 cells and proteins expression was done in HF cells.
The MSPI-p19 constructs used to express the secreted (pf19) and the anchored form (pf19+A) of the protein were generated as described previously (example 1) and contain sequences derived from *P. falciparum* (Uganda Palo-Alto isolate) sequence and a His-Tag used for the purification step. Both sequences are identical except that the one coding for the anchored form pf19+A i.e., the construct PfMSP1p19/His/GPI, includes the glycosylphosphatidylinositol (GPI) anchor signal at the C terminal end, after the His-tag.

For the protein expression, around 1.6 10⁶ HF cells/ml were infected with recombinant virus (10⁸pfu/ml; moi=10) and allowed to grow in suspension cultures, at 27°C, in Insect X-Press medium (BioWhittaker) in the presence of gentamycine, and for 2 and 3 days for pf19 and pf19+A, respectively.
The purification protocol has already been described (example 1). Briefly, the secreted form of MSP1-p19 was purified from culture supernatants, dialysed against 20mM Tris-HCl, pH8/500mM NaCl, by a passage over a 1 ml Hitrap™ chelating HP column (Amersham Pharmacia Biotech) charged with CoCl₂ 0.1M and elution with a linear gradient from 0 to 100% of 200mM imidazole in 20mM Tris-HCl, pH8/300mM NaCl, on an AKTA purifier system (Amersham Pharmacia Biotech). For the membrane bound form of MSP1-p19, purification was performed following two protocols, leading to two different immunisation trials. In each case, protein was purified in the presence of protease inhibitors (Complete EDTA-free, Roche) from the infected insect cells harvested from the culture. After solubilisation in octyl β-glucopyranoside, two purification steps were performed, one on a TALON metal affinity resin (Clontech, Palo Alto, CA) and a second, after precipitation of the contaminants in 1 M ammonium sulphate, on a Hitrap™ Octyl Sepharose FF column (Amersham Pharmacia Biotech) on a AKTA purifier system (Amersham Pharmacia Biotech). The sample was applied in 20 mM Tris HCl, pH8 containing1 M ammonium sulphate and eluted with a linear gradient from 0 to 100% of 0.05% Triton X-100 and 20 mM Tris HCl pH8 in 30% propanol-1. For the first immunisation trial (exp. 1), an additional final step of purification was performed on TALON Metal Affinity Resin as described above and eluted with 50mM imidazole in 0,05% Triton X100/20 mM Tris HCl pH8/100mM NaCl. For the second immunisation trial (exp. 2), the protein was already purified after the second purification step on the Hitrap™ Octyl Sepharose FF column and eluted at 50% of the elution buffer described above. Protein purity of each sample was checked by Coomassie blue staining 4-12% acrylamide Nu-Page gels (Invitrogen), N-terminal and mass spectrometry analysis.

### Experimental animals and subcutaneous immunization trials

Six-eight weeks old female NMRI outbred mice were obtained from Janvier (Le Genest-St-isle, France). Proteins were concentrated on Amicon Ultra (Millipore) before used in injections assays. Twenty µg of anchored MSP1-p19 (pf19+A) or secreted MSP1-p19 (pf19) were injected subcutaneously in two mouse abdomen sites. Sera from immunized animals were prepared from 100-200µl whole blood samples drawn via the tail vein.
Two immunization protocols were performed. The first (exp.1) consisted on 5 mice injected with pf19 and 5 mice with pf19+A obtained with the first purification protocol (see above). Both protein were injected in 6.4 mM Tris HCl pH8, 32 mM NaCl, 0,016% Triton X-100, 16mM imidazole. Injections were performed in the presence of Montanide ISA 51 adjuvant (SEPPIC, France) in Weight:Volume proportion. Five bleeds were performed at Day 0, Day 33, Day 110, Day 167 and Day 222, 33 days after a boost practised at Day 189 under the same protocol as the first injection. In other to evaluate the impact of Triton and imidazole on the immune response, 3 additional immunization protocol were performed in the presence of Montanide ISA51: 5 mice were injected with pf19 in PBS, 5 mice with pf19 in PBS/0.016% Triton and 5 mice with pf19 in PBS/16 mM imidazole. Two bleeds were then performed at Day 0 and Day 33 after injections.
For the second protocol (exp. 2), both proteins were injected in conditions obtained with the second purification protocol for pf19+A and final concentrations of 5 mM Tris HCl pH8, 0.006% TritonX-100. Five mice were injected with pf19 and 5 with pf19+A in the presence of Montanide ISA 51. In parallel, five mice were injected with pf19 and 5 with pf19+A in the presence of PBS (Phosphate Buffer Saline), without any adjuvant. Four bleeds were performed at days 0, 33, 70 and day 104, 33 days after a boost practised at day 71.

### ELISA

The presence of specific antibodies against purified secreted MSP1-P19 antigen as well as total levels of immunoglobulins in mouse serum samples was determined by enzyme-linked immunosorbent assay (ELISA).
*Anti-MSP1-p19 lg.* 96-well microtiter plates (Immuno-plate Maxisorp, Nunc) were coated with 0.025 µg/well of purified secreted MSP1-p19 in PBS, overnight at 4°C, and saturated with 100 µl/well 0.5% gelatine (Sigma) in PBS for 1 h. Wells were then incubated with serial dilution (starting at 1:1000 dilution for IgG, IgG1 and IgG2b detection and 1:100 dilution for IgG2a, IgG3 and IgM detection) of sera from immunized mice diluted in 0.5 gelatine/0.1 % Tween 20 (Merck)/PBS for 1h30. In order to evaluate the concentration of specific anti-MSP1 IgG2a antibodies in mg/ml, we used a mouse monoclonal anti-MSP1 IgG2a antibody name G17-12 as a standard. G17-12 hybridoma was obtained from BALB/c mice immunised with insect Sf9 cells expressing the recombinant protein MSP1-p19. G17-12 was applied at 750 ng/ml for the first dilution, and mice serum applied at serial dilutions starting at 1:100 dilution, as primary antibodies. Bound Igs were detected by the addition of a 1:6000 dilution of HRP-conjugated goat anti-mouse isotype-specific IgG and IgM-specific (Southern Biotechnology Associates Inc. (SBA), Birmingham, Alabama) or 1:7000 dilution of HRP-conjugated goat anti-murine IgG (SBA) in 0.5% gelatine/0.1 % Tween 20/PBS for 1 h. After washing, reactions were revealed adding 100 µl/well of 0.4 mg/ml o-Phenylenediamine (OPD) (Sigma) in 0.05 M phosphate-citrate buffer pH5 and 0.1% H₂O₂. After a 10 min. incubation step, the reaction was blocked with 50 µl 3 N HCl and absorbance was determined spectrophotometrically at wavelengths of 490/650 nm using a microplate reader (Molecular Devices). Titers were arbitrary defined as the maximum serum dilution used to detect a minimum O.D. of 0.5.
*Total immunoglobulin in the sera.* 96-well microtiter plates were coated overnight at 4°C with 0.375 µg/well of goat anti-mouse lg (SBA). As above, and after saturation, mice immunized serum were diluted and added at serial dilution starting at 1:1000 for 1h30. Purified mouse IgG isotypes and IgM (SBA) were also applied at 0.75 µg/ml for the first dilution and used as standard and antibody detection was then carried out as described above.

### 2-2-Results

### 1) Both proteins are highly immunogenic

No systemic or local reactions were detected after any of the immunization assays, indicating that all antigens as well as the adjuvant are well tolerated by the mice. The minor differences detected between individual mice could be attributed firstly, to the fact that they are out-bred mice and secondly to variations in age as they arrived at the laboratory between 6 and 8 weeks old. However, no difference in level was detected between groups injected with pf19 or pf19+A, in both trial 1 or 2 and for each IgG isotypes and IgM. *Non-specific antibodies detection.* At day 0, in all groups, mice had more IgG than IgM, and less represented IgG isotype was IgG3 (Mean of all groups for both experiments: 0.046 mg/ml (n=30, range: 0.011-0.103)) when IgG2b (Mean of all groups for both experiments: 0.41 mg/ml (n=30, range: 0.163-0.955)) was the most represented one. Concentrations of total antibodies of each mouse detected before and 167 days after immunizations in the first trial are presented in Figure 1 and in Table 3A.

**Table 3**. Evaluation of the immune response generated after immunisation with the anchored (GPI-MSP1-19) or the
secreted (MSP1-19) form of MSP1-19: ratio of total serum antibodies induced in:
(A) vaccine trial 1: 167 days after the injection and 33 days after a second injection performed on day 189 (day 222)
of each protein diluted in 6.4mM Tris pH8, 32mM Nacl, 0.016% Triton X-100, 16mM imidazole and in the presence of
Montanide ISA 51.
(B) vaccine trial 2: 33 days after the injection of each protein diluted in 5mM Tris pH8, 0.006% Triton X-100, in the
presence (Montanide ISA 51) or in the absence (PBS) of adjuvant.
Each result corresponds to the mean of 5 mice.

| **A** | | | | | | |
|---|---|---|---|---|---|---|
| | GPI-MSP1-19 | | MSP1-19 | | ratio GPI-MSP1-19/ MSP1-19 Day 167* | ratio GPI-MSP1-19/ MSP1-19 Day 222** |
| | ratio D167/D0 | ratio D222/D0 | ratio D167/D0 | ratio D222/D0 | | |
| IgG | 6.1 | 9.7 | 3.0 | 7.3 | 2.0 | 1.3 |
| IgG1 | 7.9 | 12.3 | 4.8 | 8.2 | 1.6 | 1.5 |
| IgG2a | 7.5 | 14.0 | 7.9 | 8.3 | 1.0 | 1.7 |
| IgG2b | 3.5 | 6.3 | 3.3 | 6.9 | 1.0 | 0.9 |
| IgG3 | 12.9 | 8.1 | 5.8 | 4.8 | 2.2 | 1.7 |
| IgM | 3.8 | 3.5 | 3.6 | 5.0 | 1.1 | 0.7 |
| *ratio GPI-MSP1-19/MSP1-19 Day 167=(ratio D167/D0)GPI-MSP1-19/(ratio D167/D0)MSP1-19 | | | | | | |
| **ratio GPI-MSP1-19/MSP1-19 Day 222=(ratio D222/D0)GPI-MSP1-19/(ratio D222/D0)MSP1-19 | | | | | | |

| **B** | | | | | | |
|---|---|---|---|---|---|---|
| | Montanide ISA 51 | | | PBS | | |
| | ratio GPI-MSPI-19 D33/D0 | ratio MSPI-19 D33/D0 | ratio GPI-MSPI-19/ MSP1-19* | ratio GPI-MSPI-19 D33/D0 | ratio MSPI-19 D33/D0 | ratio GPI-MSPI-19/ MSP1-19* |
| IgG | 2.3 | 2.1 | 1.1 | 1.6 | 1.6 | 1 |
| IgG1 | 5.2 | 3.3 | 1.6 | 1.9 | 1.1 | 1.7 |
| IgG2a | 1.5 | 3.0 | 0.5 | 5.6 | 2.3 | 2.4 |
| IgG2b | 1.5 | 1.5 | 1,0 | 1.3 | 1.6 | 0.8 |
| IgG3 | 3.3 | 7.2 | 0.5 | 4.0 | 3.4 | 1.2 |
| IgM | 2.1 | 2.6 | 0.8 | 1.9 | 1.4 | 1.3 |
| *ratio GPI-MSP1-19/MSP1-19=(ratio D33/D0)GPI-MSP1-19/(ratio D33/D0)MSP1-19 | | | | | | |

Immunizations with both proteins lead to an increase of all antibody subclass level, with the largest proportion of antibodies being of the IgG1 isotype (mean of 1.51 mg/ml and 1.33 mg/ml for pf19+A and pf19, respectively). The increase of the total IgG corresponded to 6-fold and 3-fold for pf19+A and pf19, respectively, and, in each group, the highest increase concerned the lgG3 (13-fold rises) for pf19+A and the lgG2a (8 fold rises) for pf19 (Table 3). The same general isotype profile was recovered before and 33 days after the boost, at day 222 (Fig.1C, Table 3A). At this date, the induction of total IgG antibodies was around 2 times higher than at Day 167 for both proteins with the exception of IgG3. Looking at IgM levels, a slight increase was detected for the secreted protein and and a slight decrease for the anchored one.
Figure 2A and Table 3B show that, in trial 2, all IgG isotypes increased 33 days after immunisation with pf19+A or pf19 in the presence of Montanide, and that if global antibody levels were less than those detected at Day 167 in the first trial, they were in agreement with the previously seen domination of the IgG1 isotype (mean of 1.65 mg/ml and 1.25 mg/ml for pf19+A and pf19, respectively). The increase of the total IgG corresponded to more than 2-fold for both proteins, and IgG1 displayed the highest increase (5.2-fold rises) for pf19+A while it was IgG3 (7.2-fold rises) for pf19. This last result has to be considered carefully because such an significant IgG3 increase for the secreted protein came from one mouse among the 5 tested, which had a very high IgG3 titer at day 33. In addition, the high increase concerning the IgG2a was essentially due to the fact that 3 mice out of 5 had no detectable IgG2a antibodies at day 0 in this group.
Immunisation with protein without adjuvant induced IgG responses, which exhibited same isotype profile as observed with Montanide and showing that the proteins themselves were capable to generate a general immune response. The increase in IgG was 1.4 and 1.3 times weaker than after immunisation in the presence of the adjuvant for pf19+A and pf19, respectively. The highest increase concerned the IgG2a for pf19+A followed by IgG3 isotypes for both proteins (Fig. 2B, Table 3B).

*Specific antibodies detection.* Previous experiments have shown that injection of only Montanide ISA51 without antigen did not induced any anti-MSP1-p19 antibodies (Data not shown). As expected, no mice had any anti-MSP1-p19 antibodies at the beginning of each trial (J0), before the injection of the antigens.
Concerning the trial 1, a single s.c. injection of 20µg of pf19 or pf19+A induced high titers of serum anti-MSP1 antibodies, which were maintained at a steady level until Day 167 (Fig 3). Even if it is difficult to compare the titers of each antibody class and subclasses because of potential misinterpretation that may result from widely different affinities of the secondary antibodies, we can estimate, with the first serum dilution used (1 :1000), that the anti-MSP1-P19 response seemed to be essentially of the IgG1 types for each protein (estimated maximum titer 33 day days after a boost: 1/1000000) . Concerning the IgG2a and IgG2b, the interpretation is difficult because of the cross-reactivity that exists between the two subclasses, but lgG2b seems to be the second most representative subclass (first dilution serum used: 1:1000 and estimated maximum titer 1/145000). Anti-MSP1 IgM antibodies were near the lowest level that could be detected in any serum and at any of the bleeds, which is a normal thing at day 33 post-injection. The same isotype subclass profile was recovered at days 33, 110, 167, as well as after a second injection. This boost led to an increase in the level of all IgG isotype antibodies specific to MSP1-P19 except IgG3.
Data obtained from trial 2 confirmed the induction of a strong IgG anti-MSP1-P19 response until 70 days after the immunisation with pf19+A or pf19 in the presence of Montanide, and a predominance of the IgG1 subclass (Fig 4A), though slightly less rapid than observed in trial 1 but of the same intensity at day 70 (estimated tier: 1/730000). When each protein was injected without adjuvant a specific, while weak, anti-MSP1-p19 response was only detected with pf19+A (also predominantly of the IgG1 type with estimated titer of 8000 at day 70 after only one injection) (Fig 4B).

**Table 4:**

| | | GP1-MSP1-19 | | | MSP1-19 | | | ratio | ratio |
|---|---|---|---|---|---|---|---|---|---|
| | | anti-MSPI-19 IgG2a mg/ml | total IgG2a mg/ml | ratio %* | anti-MSPI-19 IgG2a mg/ml | total IgG2a mg/ml | ratio %* | GPI-MSPI-19/MSP1-19 | GPI-MSPI-19/ratio MSP1-19* |
| | | | | | | | | | |
| Trial 1 | D0 | 0 | 0.08 | 0 | 0 | 0.12 | 0 | | |
| | D167 | 0.088 | 0.62 | 16.3 | 0.037 | 0.97 | 4.3 | 2.4 | 3.8 |
| | D222 | 0.221 | 1.15 | 20.45 | 0.049 | 1.02 | 5.4 | 4.5 | 3.8 |
| | | | | | | | | | |
| Trial 2 | D0 | 0 | 0.2 | 0 | 0 | 0.05 | 0 | | |
| | D33 | 0.019 | 0.3 | 19 | 0.003 | 0.151 | 2.3 | 6.3 | 8.3 |

| | | | | | | | | | |
|---|---|---|---|---|---|---|---|---|---|
| * relative proportion of specific antibodies among total induced antibodies for each antigen | | | | | | | | | |

As summarised in Table 4, MSP1-P19 specific IgG2a were absent in all mice at Day 0. At Day 167 of the first trial, quantification of IgG2a revealed that 16.3% of the IgG2a produced after immunization with pf19+A and 4.3% of IgG2a produced after immunization with pf19 were some anti-MSP1-P19-IgG2a. Result obtained after the second injection show a 2.5-fold and 1.3-fold rises in anti-MSP1-P19 IgG2a between Day 167 and 33 days after boosting for pf19+A and pf19, respectively. The percentage of IgG2a antibodies specific to MSP1-P19 among total ones increased after boosting to 20.45% and 5.4% for pf19+A and pf19, respectively, confirming the specific action of the antigen. During the trial 2 with Montanide, the percentage of anti-MSP1-P19-IgG2a among total induced IgG2a, was almost identical with 19% for pf19+A and 2.3% for pf19. Such results indicated that both proteins generated a polyclonal response in addition to a specific one, at least as far as this isotype is concerned. It is noteworthy that, since only non-specific IgM was detected, a degree of general immune stimulation has occurred, indicating a possible additional stimulation by antigens present in the animal laboratory environment. However, it is also important to specify here that we evaluated only antibodies against MSP1-P19 as we have coated the secreted form of the protein and that the possible antibodies against the C-terminal part of the anchored form were not taken into account.

### 2) Effect of GPI on the immune response

*Non-specific antibodies detection.* When comparing the level of total IgG 167 days after an immunisation with Pf19+A or with Pf19 in the presence of Montanide (trial 1), we showed that levels are augmented 2-fold in the presence of the GPI form of the protein. This increase is 2.2-fold and 1.6-fold higher for the IgG3 and IgG1, respectively, and is comparable for the IgG2a, IgG2b and IgM antibodies (Fig.1, Table 3A). After a second injection, comparable isotype profiles were recovered except a difference that emerges for the lgG2a antibodies, with a 1.7-fold higher induction with Pf19+A than with Pf19.
This general IgG subclasses repartition was not completely recovered 33 days after immunizations performed with the adjuvant in the trial 2 (Fig 2A, Table 3B). The induction of lgG1 antibodies remained 1.6 times higher with ph19+A than with pf19. After the lgG1, lgG3 remained the highest increase for the anchored protein (3.4-fold rises) but, for this isotype as well as for IgG2a and IgM, we found a higher increase with pf19 but with the restriction discussed above.
Immunisation of each protein in the absence of adjuvant showed that if no difference was detected concerning the increase of total IgG, a higher level of increase of IgG1 (1.7-fold rises) and IgG2a (2.4-fold rises) was detected with pf19+A (Fig. 2B, Table 3B) while an almost equivalent level was detected concerning the other isotypes.

*Specific antibodies detection.* Concerning the anti-MSP1-P19 antibodies, immunization with pf19+A in the presence of Montanide leads to higher titers of IgG3 and IgG2a than with pf19, 33, 110, 167 days after the injection (trial 1 and 2), as well as 33 days after the second injection in the trial 1. Titers were almost similar concerning the IgG1 and IgG2b between the two proteins with only slight higher titers with pf19+A (Fig. 3 and 4A). Injection of both proteins without adjuvant showed that 33 days later, some IgG1, IgG2a, IgG2b and, in a lesser extent, IgG3 are detected with pf19+A when no antibodies could be detected for pf19 even with a serum dilution of 1:100 (Fig 4B).
While no difference was detected between the two proteins concerning the total IgG2a antibodies after one injection (see Table 3), results obtained with the use of Mab G17-12 as standard (Table 4) have confirmed that, concerning the anti-MSP1-P19 IgG2a, 2.4-fold more antibodies were produced after injection with pf19+A as compared to pf19 at day 167 of the trial 1. After the second injection, this difference increased with 4.5-fold more IgG2a concerning pf19+A. In addition, the proportion of specific anti-MSP1-P19 IgG2a antibodies among the total induced IgG2a antibodies was 3.8 times higher after pf19+A injection (either one or two) than after pf19 injection. Concerning the second trial in the presence of adjuvant, we found 6 and 5-fold more anti-MSP1-P19 lgG2a antibodies 33 and 70 days, respectively after injection with pf19+A than with pf19, and 8.3-fold more specific IgG2a among total induced ones with pf19+A than with pf19. In the absence of adjuvant, no IgG2a were detectable for pf19 while, for pf19+A, 20-fold less IgG2a were detectable 70 days after the injection (0.002 mg/ml) by comparison with the injection performed in the presence of ISA 51.

### 4) Imidazole and Triton effect

To determine whether the imidazole or the Triton used during the trial 1 played a role in the induction of the high antibodies titers or isotypes obtained, we performed 3 series of mice immunisation with the secreted form of MSP1-p19 in the presence of adjuvant Montanide, with 16mM imidazole, 0,016% Triton or PBS only. Concerning the non-specific response the ratios between antibody concentrations estimated at Day 33 on those detected on day 0 were equivalent with each formulation, indicating that there is no impact of Triton or imidazole on the non specific immune response (Data not shown). No anti-MSP1-P19 antibodies were detected at Day O. Thirty-three days after the immunisation, no specific IgM were detected and all IgG MSP1-P19 specific antibodies detected showed equivalent titres for each group (data not shown). Therefore, no influence of imidazole or Triton was detected on the immune response against MSP1-P19, when used in the concentrations of the first trial.

### 3-3-Discussion

Two series of vaccination trials were performed in order to determine the immunogenicity of baculovirus recombinant protein MSP1-p19 produced in order to obtain 2 forms of the protein: a GPI-anchored one and a secreted one. Expression and purification of such proteins have been already described (Bonnet, submitted). Constructions coding for each protein were similar except that the one designed to be GPI-anchored has the signal sequence coding for the GPI attachment at the C-terminal end. As expected, the absence of the GPI anchor sequence resulted in the failure of the protein to attach to the insect cell membrane and she was secreted in the culture medium, when the presence of such a structure permitted to obtain a membrane bound protein. We have showed that the insect expression system can add properly a GPI anchor to MSP1-P19 and that both form of the protein were recognized by antibodies present in human immune serum from endemic country, suggested that their conformation represents that of the native antigen MSP1-P19 (Bonnet, submitted).Both form, anchored (Pf19+A) and secreted (pf19), of the protein MSP1-p19 were highly immunogenic in mice and generate anti-MSP1-P19 antibody responses predominantly, but not exclusively, of the cytophilic IgG1 subclass. The use of a monoclonal antibody as a standard for anti-MSP1-IgG2a as well as the evaluation of the total antibodies in mice serum has permitted us to establish that both proteins induce specific as well as non-specific humoral responses. In adjuvant formulation, both proteins stimulate comparable proportions of specific IgG1, IgG2b and IgM. Some difference was observed concerning anti-MSP1-P19 IgG2a, with more antibodies induced by the GPI form of the protein. Finally, a greater and clear difference was recovered concerning the anti-MSP1-P19 IgG3 with a highest proportion of this isotype after an immunisation with Pf19+A than with pf19. These differences were reproducible between trials. Finally, we demonstrated that, in the absence of any adjuvant, pf19+A is capable to induce a specific immune response while pf19 does not.

### 1) Proteins immunogenicity

It is interesting to note that on one hand, the antibody responses to both antigens are predominantly an IgG1 response, which represents classically a T-cell dependent response and, on the other hand, the both antigens stimulated also predominantly lgG2b, which corresponds usually to a T-cell independent response. Although similarities exist for many antigens within an antigen class, the several exceptions suggest that no single feature of antigen structure regulates isotype preference [69]. So it is clear that the categorization of antigens into subclasses of T-dependent or independent cells although operationally useful, implies special properties of the antigen and that some intermediate pattern such as those observed for MSP1-p19 exist. In addition, results indicated that both T-helper 1 (as indicated by the induction of lgG2a) and T-helper 2 (as indicated by the induction of lgG1) subsets of T-helper cells were elicited by vaccination with both MSP1-P19 protein forms, which is considered as a primordial thing for an effective anti-malaria vaccine [70]; [71]. The fact that we observed a T-cell dependant response with recombinant MSP1-P19 from *P. falciparum* is in favour of the use of such part of the MSP1 protein as a malaria vaccine, which is not in agreement with previous studies claiming that immunodominant T-cell epitopes were found in sequences of the MSP1-33 region and not in the MSP1-P19 one [25]; [72]; [73].
The immune response obtained after immunisation with the two proteins exhibited very high specific antibody titres, which were maintained for more than 5 months with only one injection. Dosage of the total antibodies showed as well that the global immune response was increased more than 6-fold if we consider for example the total IgG generated 167 days after an injection with the GPI form of the protein. Although the production of antibodies recognizing MSP1-P19 epitopes does not represents the majority of antibodies induced by the secreted or the anchored form of the protein, we showed that, in trial 1 and at least concerning the IgG2a, from 16% (after one injection) to 20% (after two injections) of these antibodies were some specific ones after immunization with the anchored protein form, while theses percentages were only 4.3 to 5.4% with the secreted protein form.
Antibodies induced by both proteins were predominantly of the IgG1 type followed by IgG2b and IgG2a types. This isotype profile is similar to those observed by Dutta *et al.* after mice immunization with *P. vivax* MSP1-42 expressed in *Escherichia coli* [74]. The murine cytophilic IgG1 and IgG2a antibodies have been implicated in protection against murine malaria in several studies. Even, in 1978, green *et al.* demonstrated that the cytophilic immunoglobulin G1 was protective against *P. berghei* in rat. After that, in 1987, Falanga *et al.* showed that, in immunized mice, protected against a parasite challenge, the isotypic pattern showed that the lgG1 and IgM constituting the main part of the response [75]. Concerning lgG2a antibodies, it was also demonstrated that, in mice, they play dominant role in mediating immunity to *P. yoeli* [76], *P. chabaudi* [77]; [78] and *P. berghei* [79]. A recent study has confirmed such results and showed that, in mice, the sequential upregulated synthesis of IgG2a and IgG1, in that order, was associated with protective immunity against *P. yoeli* and *P. chabaudi* infections [80].

The use of adjuvant is a very important approach to enhancing the immunogenicity of vaccines and some new impressive adjuvants are urgently needed for human use. Our results shown that the Montanide ISA 51, which is generally considered safe for human use, is a powerful immunostimulator, which can be envisaged for making single dose vaccines. This adjuvant is an oil and water emulsion and its formulation delays the degradation of the antigen and also provides controlled slow release of antigen, which probably participate in the high and long lasting immuno-stimulation reflected in our results.

### 2) Difference between the two proteins (with and without anchor sequence)

Purified antigens are not usually strongly immunogenic on their own and require the addition of adjuvant. The injection of the both proteins forms without any adjuvant generated effectively a general immune response where around 1.5-time less total antibodies were detected than with the adjuvant. However, results showed that only the anchored form of the protein could induce a specific immune response without any adjuvant, even if this response was 20 time weaker than with the adjuvant as far as the anti-MSP1 lgG2a are concern. Such a result represents a critical determinant for its use in vaccinology. These data suggest that the higher immune response detected with this protein has to be attributed to the protein and not to a different presentation of this antigen by the adjuvant. In addition, differences recovered after an immunization with the anchored form of the protein has to be attributed to the additional C-terminal part of the protein including GPI structures, as it is the only difference between the 2 recombinant proteins. To our knowledge, it is the first demonstration that a GPI structure can act itself as an adjuvant and can be used with antigens that carry their own covalent immunostimulator and/or immunomodulator. Our result showed effectively that GPI interacts with the immune system to enhance the immune response but as well to specifically modify the isotype of the response to the given antigen. It is possible that the two proteins stimulate the same subpopulations of B cells, essentially restricted to IgG1 and that the GPI form of the protein stimulate, in addition, a second subpopulation of B cells, restricted for example to IgG3 via the C terminal additional part of the protein corresponding to the glycolipidic GPI structure. It was effectively establish that anti-soluble protein or anti-carbohydrate antibodies are generally restricted to the lgG1 and lgG3, respectively [81]; [82]. As we have only 14% of GPI anchored protein in our pf19+A preparation (the other corresponding to non-processed protein either with the signal peptide or the sequence coding for the GPI attachment) it is possible that other forms of the protein present in this preparation were responsible of the resulting immune response by stimulating a panel of several subpopulations of B cells. This raises the possibility that antigens select IgG subclasses on the basis of selective interaction with B-cell receptors as suggested by [69]. It is noteworthy that *in vitro* study has showed that GPI-related glycoinositolphospholipids from *Trypanosoma cruzi* can induce both IgG3 and lgG1 production by murine B cells, in the absence of covalently linked proteins but with a requirement for cytokine costimulation [83].

GPI anchors from parasitic protozoa have been shown to exert a wide variety of effects on cells of the host innate immune system both by stimulating/regulating cells in a non-specific manner and by serving as targets for both specific T and B lymphocytes responses (review in [84]). However, the receptor(s) that are triggered by GPls has not been identified and several proposition have been made. Joyce *et al.* have demonstrated that GPI anchors are the main natural ligand associated with mouse CD1d molecules [85] and Schofield *et al.* have demonstrated that NK cells were stimulated by GPls in a CD1d-dependant manner [33]. However, such results were controversial and, in 2000, a study showed that, the absence of CD1d expression in knockout mice does not significantly influence the titers of IgGs directed against the GPI-anchored CS protein of *P. berghei,* and that B cell help is mediated through classical MHC class II/CD4+ T cell interaction [86]. Some studies have also demonstrated that GPI from *Trypanosoma cruzi* bind to CD1d but without eliciting dominant innate or adaptative immune response via this pathway [87], while previous ones reported that *T. cruzi* GPls might stimulate NK T cell in a CD1 d-restricted manner [88]. On the other hand, GPI of this protozoon have been implicated in the activation of Tool Like Receptor 2 (TLR-2) from both mouse and human origin, activation that lead to the initiation of the various macrophage functions induced by the GPIs [89]; [90]. Recently it was also demonstrated that the adjuvanticity of a TLR ligand, the Macrophage-activating lipopeptide-2 (MAP-2) from *Mycoplasma fermentans,* can be mediated, at least in part, by the stimulation of Dendritic cells maturation [91]. Consequently, TLR-ligands have been proposed as new possible vaccine adjuvants [92].
The questions if the insect-GPI generated adjuvant signal observed here implicates Toll like receptors or CD1 d and the activation of macrophages as well as dendritic cells, is now open. *In vivo* immunisation of mice that lack CD1d or TLR with both recombinant MSP1-P19 protein forms should answer questions related to this pathway. The fact that these both receptors implicated in activation of the innate immunity are unrestricted emphasise the potential for GPIs as future constituents of vaccines. In fact, if GPls are TLR or CD1d ligands, the low polymorphism detected for these receptors let us to envisage that GPIs could generate equal adjuvant signal for everyone without any HLA restriction. Naturally, a key feature for using insect-GPI as adjuvant will be its safety in human, which should first has to be tested in non human primates.

Our results showed that Pf19 and pf19+A antigens stimulated different patterns of response especially as far as the IgG2a was concerned, with 2.4 to 6.3-fold more IgG2a induced with the anchored MSP1-P19 form of the protein. Such IgG subclass is generally induced by viral infections [93] but also by parasite infections [79]; [94]. IgG2a activates the complement system more readily than do tgG1 antibodies [95] and are quite efficient mediators of antibody-dependant cell-mediated cytotoxicity [96]. They bond to specific receptors that are expressed on murine macrophages and are involved in phagocytosis [97]. It was demonstrated that such antibody subclass is elicited by interferon-gamma [98]; [99]. As previously mentioned, the fact that IgG2a have been implicated in the protection against murine malaria is a positive point concerning the use of a GPI-protein in vaccinology against malaria.

The GPI form of MSP1-P19 also induces significantly more total as well as specific IgG3 than the secreted form of the protein. Classically, IgG3 constitutes a very low fraction of IgG responses to T cell-dependent protein Ag [81]; [100]. It was demonstrated a long time ago that antigens can be categorized according to which of the four mice IgG subclasses is elicited [101] and post-translational modification of proteins by lipids and carbohydrates are well known to influence the antigen presenting cell type and thus antibody subclass expression [81]; [102]; [93]. The specificity of IgG3 is directed primarily against carbohydrates, polysaccharides and repeating epitope Ags [81]; [103]. The IgG3, found in normal mouse serum in very low amounts, appear early in immune responses independently of T cell help and, as such, are early effector molecules of the immune system which can elicit powerful effector function like complement activation [81]; [103]. It has been shown that IgG3 antibodies are important in the resistance of pathogens like *Streptococcus pneumoniae* [104]; [105] or *Candida albicans* [106]. In 1998, Gavin *et al.* have identified the mouse IgG3 macrophage receptor as FcγRI, which was confirmed by recent data [107]; [108]. It was demonstrated that IL10 selectively up-regulated IgM to IgG3 class switching [109] and that NK cells, which are a source of IL10, may play a role in mediating such humoral immunity [110].

### 3) Human immunity

Human immunity to malaria is quite complex and still not completely understood but it now appears clear that humoral immune mechanisms may be very important in controlling the blood stages of the parasite. In this context of human malaria, the association between isotypes distribution and protective immunity remains controversial. However, it seems to emerge that Antibody-dependent protection against *P. falciparum* is primarily mediated by human cytophilic IgG antibodies (human IgG1 and IgG3), which activate cytotoxic and phagocytic effector functions of neutrophils and monocytes [111]; [112]; [113]; [114]; [115]. Thus, such a proposition correlates with the fact that murine cytophilic IgG1 and IgG2a antibodies are protective against malaria infection in mice, suggesting the necessity of a immune response implicating a Th1 as well as a Th2 response. In addition, Yount et al first demonstrated that polysaccharide but not protein antigens stimulated preferentially human IgG2 subclass [116] and this result was confirmed for a variety of polysaccharides [117]; [118]; [119]. The question if human IgG2 and mice IgG3 share a common ancestry have been raised as human IgG2 is the subclass associated with many anticarbohydrate variable regions and is the one supposed to be the most primitive of the human IgG subclasses [120]; [119]. However, during a fiels study performed in Papua New Guinea, Boutlis *et al.,* have determined that the antibody response induced by *P. falciparum* GPls is characterized predominantly by lgG3, with a lesser contribution from lgG1 and an absence of lgG2 and lgG4.

### Conclusion

In conclusion, our study demonstrates that the baculovirus system is a powerful expression system for producing highly immunogenic recombinant malaria proteins and that GPI-posttranscriptional modification of these proteins are implicated in the resulting immune response. The question if it is possible or not to generate some anti-toxic antibodies against *P. falciparum* GPls by vaccination with insect GPls needs to be tested. However, to our point of view, this is not the most promising advantage of integrating an insect GPI anchor in a malaria vaccine. The most interesting thing is the modulated immune response generated by this GPI anchor against the protein antigen MSP1-P19 and to see whether this structure can also act as an adjuvant for any other proteins. It will be now of great interest to provide insights into signalling events that are mediated by the presence of a GPI covalently linked to a recombinant protein produced in the baculovirus system. As well, the question whether the observed switching in antibody isotypes in the presence of the GPI is a good thing or not is now open and it would be also of great interest to test this antigen in monkey models in order to evaluated the protective effect of such elevated IgG3 and IgG2a antibodies.

### References

1. Gerold, P., et al., Structural analysis of the glycosyl-phosphatidylinositol membrane anchor of the merozoite surface proteins-1 and -2 of Plasmodium falciparum. Mol Biochem Parasitol, 1996. 75(2): p. 131-43.
2. Blackman, M.J., et al., A single fragment of a malaria merozoite surface protein remains on the parasite during red cell invasion and is the target of invasion- inhibiting antibodies. J Exp Med, 1990. 172(1): p. 379-82.
3. Holder, A.A. and M.J. Blackman, What is the Function of Msp-I on the Malaria Merozoite. Parasitology Today, 1994. 10(5): p. 182-184.
4. O'Donnell, R.A., et al., Functional conservation of the malaria vaccine antigen MSP-119across distantly related Plasmodium species. Nat Med, 2000. 6(1): p. 91-5.
5. Hughes, M.K. and A.L. Hughes, Natural selection on Plasmodium surface proteins. Mol Biochem Parasitol, 1995. 71(1): p. 99-113.
6. Jongwutiwes, S., K. Tanabe, and H. Kanbara, Sequence conservation in the C-terminal part of the precursor to the major merozoite surface proteins (MSP1) of Plasmodium falciparum from field isolates. Mol Biochem Parasitol, 1993.59(1): p. 95-100.
7. Kang, Y. and C.A. Long, Sequence heterogeneity of the C-terminal, Cys-rich region of the merozoite surface protein-1 (MSP-1) in field samples of Plasmodium falciparum. Mol Biochem Parasitol, 1995. 73(1-2): p. 103-10.
8. Kaneko, O., et al., Plasmodium falciparum: allelic variation in the merozoite surface protein 1 gene in wild isolates from southern Vietnam. Exp Parasitol, 1997. 86(1): p. 45-57.
9. Qari, S.H., et al., Predicted and observed alleles of Plasmodium falciparum merozoite surface protein-1 (MSP-1), a potential malaria vaccine antigen. Mol Biochem Parasitol, 1998. 92(2): p. 241-52.
10. Chitarra, V., et al., The crystal structure of C-terminal merozoite surface protein 1 at 1.8 A resolution, a highly protective malaria vaccine candidate. Mol Cell, 1999. 3(4): p. 457-64.
11. Pizarro, J.C., et al., Crystal structure of a Fab complex formed with PfMSP1-19, the C-terminal fragment of merozoite surface protein 1 from Plasmodium falciparum: a malaria vaccine candidate. J Mol Biol, 2003. 328(5): p. 1091-103.
12. Riley, E.M., et al., Naturally acquired cellular and humoral immune responses to the major merozoite surface antigen (PfMSP1) of Plasmodium falciparum are associated with reduced malaria morbidity. Parasite Immunol, 1992. 14(3): p. 321-37.
13. Egan, A.F., et al., Clinical immunity to Plasmodium falciparum malaria is associated with serum antibodies to the 19-kDa C-terminal fragment of the merozoite surface antigen, PfMSP-1. J Infect Dis, 1996. 173(3): p. 765-9.
14. Shi, Y.P., et al., Natural immune response to the C-terminal 19-kilodalton domain of Plasmodium falciparum merozoite surface protein 1. Infect Immun, 1996. 64(7): p. 2716-23.
15. Shai, S., M.J. Blackman, and A.A. Holder, Epitopes in the 19kDa fragment of the Plasmodium falciparum major merozoite surface protein-1 (PfMSP-1(19)) recognized by human antibodies. Parasite Immunol, 1995. 17(5): p. 269-75.
16. Branch, O.H., et al., A longitudinal investigation of IgG and IgM antibody responses to the merozoite surface protein-1 19-kiloDalton domain of Plasmodium falciparum in pregnant women and infants: associations with febrile illness, parasitemia, and anemia. Am J Trop Med Hyg, 1998. 58(2): p. 211-9.
17. Perraut, R., et al., Distinct surrogate markers for protection against Plasmodium falciparum infection and clinical malaria identified in a Senegalese community after radical drug cure. J Infect Dis, 2003. 188(12): p. 1940-50.
18. Egan, A.F., et al., Human antibodies to the 19kDa C-terminal fragment of Plasmodium falciparum merozoite surface protein 1 inhibit parasite growth in vitro. Parasite Immunol, 1999. 21(3): p. 133-9.
19. O'Donnell, R.A., et al., Antibodies against merozoite surface protein (msp)-1(19) are a major component of the invasion-inhibitory response in individuals immune to malaria. J Exp Med, 2001. 193(12): p. 1403-12.
20. Kumar, S., et al., Immunogenicity and in vivo efficacy of recombinant Plasmodium falciparum merozoite surface protein-1 in Aotus monkeys. Mol Med, 1995. 1 (3): p. 325-32.
21. Burghaus, P.A., et al., Immunization of Aotus nancymai with recombinant C terminus of Plasmodium falciparum merozoite surface protein 1 in liposomes and alum adjuvant does not induce protection against a challenge infection. Infect Immun, 1996. 64(9): p. 3614-9.
22. Kumar, S., et al., Immunogenicity and efficacy in aotus monkeys of four recombinant Plasmodium falciparum vaccines in multiple adjuvant formulations based on the 19-kilodalton C terminus of merozoite surface protein 1. Infect Immun, 2000. 68(4): p. 2215-23.
23. Egan, A.F., M.J. Blackman, and D.C. Kaslow, Vaccine efficacy of recombinant Plasmodium falciparum merozoite surface protein 1 in malaria-naive, -exposed, and/or -rechallenged Aotus vociferans monkeys. Infect Immun, 2000. 68(3): p. 1418-27.
24. Stowers, A.W., et al., Efficacy of Two Alternate Vaccines Based on Plasmodium falciparum Merozoite Surface Protein 1 in an Aotus Challenge Trial. Infect Immun, 2001. 69(3): p. 1536-1546.
25. Chang, S.P., et al., A recombinant Baculovirus 42-kilodalton c-terminal fragment of Plasmodium falciparum merozoite surface protein 1 protects Aotus monkeys against malaria. Infection and Immunity, 1996. 64(1): p. 253-261.
26. Perera, K.L., et al., Baculovirus merozoite surface protein 1 C-terminal recombinant antigens are highly protective in a natural primate model for human Plasmodium vivax malaria. Infect Immun, 1998. 66(4): p. 1500-6.
27. Stowers, A.W., et al., A recombinant vaccine expressed in the milk of transgenic mice protects Aotus monkeys from a lethal challenge with Plasmodium falciparum. Proc Natl Acad Sci U S A, 2002. 99(1): p. 339-44.
28. Ferguson, M.A., et al., Glycosyl-phosphatidylinositol molecules of the parasite and the host. Parasitology, 1994. 108 Suppl: p. S45-54.
29. Schofield, L. and S.D. Tachado, Regulation of host cell function by glycosylphosphatidylinositols of the parasitic protozoa. Immunol Cell Biol, 1996a. 74(6): p. 555-63.
30. Schofield, L. and F. Hackett, Signal transduction in host cells by a glycosylphosphatidylinositol toxin of malaria parasites. J Exp Med, 1993. 177(1): p. 145-53.
31. Tachado, S.D., et al., Glycosylphosphatidylinositol toxin of Plasmodium induces nitric oxide synthase expression in macrophages and vascular endothelial cells by a protein tyrosine kinase-dependent and protein kinase C-dependent signaling pathway. J Immunol, 1996. 156(5): p. 1897-1907.
32. Schofield, L., et al., Glycosylphosphatidylinositol toxin of Plasmodium up-regulates intercellular adhesion molecule-1, vascular cell adhesion molecule-1, and E-selectin expression in vascular endothelial cells and increases leukocyte and parasite cytoadherence via tyrosine kinase-dependent signal transduction. J Immunol, 1996b. 156(5): p. 1886-96.
33. Schofield, L., et al., CD1d-restricted immunoglobulin G formation to GPI-anchored antigens mediated by NKT cells. Science, 1999. 283(5399): p. 225-9.
34. Naik, R.S., et al., Glycosylphosphatidylinositol anchors of Plasmodium falciparum: molecular characterization and naturally elicited antibody response that may provide immunity to malaria pathogenesis. J Exp Med, 2000b. 192(11): p. 1563-76.
35. de Souza, J.B., et al., Prevalence and boosting of antibodies to Plasmodium falciparum glycosylphosphatidylinositols and evaluation of their association with protection from mild and severe clinical malaria. Infect Immun, 2002. 70(9): p. 5045-51.
36. Boutlis, C.S., et al., Antibodies to Plasmodium falciparum Glycosylphosphatidylinositols: Inverse Association with Tolerance of Parasitemia in Papua New Guinean Children and Adults. Infect Immun, 2002. 70(9): p. 5052-5057.
37. Schofield, L., et al., Synthetic GPI as a candidate anti-toxic vaccine in a model of malaria. Nature, 2002. 418(6899): p. 785-9.
38. Longacre, S., K.N. Mendis, and P.H. David, Plasmodium vivax merozoite surface protein 1 C-terminal recombinant proteins in baculovirus. Mol Biochem Parasitol, 1994. 64(2): p. 191-205.
39. Chang, S.P., et al., Plasmodium falciparum: gene structure and hydropathy profile of the major merozoite surface antigen (gp 195) of the Uganda-Palo Alto isolate. Exp Parasitol, 1988. 67(1): p. 1-11.
40. Summers, M.D. and G.E. Smith, A manual of methods for baculovirus vectors and insect cells culture procedures. Texas Agric. Exp. Station bull., 1987. 1555.
41. Fergusson, M.A.J., in Lipid modification of proteins. A practical approach., A.J. Turner and N.M. Hooper, Editors. 1992, IRL press: Oxford. p. 191-230.
42. Kamerling, J.P., et al., Characterization by gas-liquid chromatography-mass spectrometry and proton-magnetic-resonance spectroscopy of pertrimethylsilyl methyl glycosides obtained in the methanolysis of glycoproteins and glycopeptides. Biochem J, 1975. 151 (3): p. 491-5.
43. Nagarajan, S. and P. Selvaraj, Expression and characterization of glycolipid-anchored B7-1 (CD80) from baculovirus-infected insect cells: protein transfer onto tumor cells. Protein Expr Purif, 1999. 17(2): p. 273-81.
44. Brown, D.A. and J.K. Rose, Sorting of GPI-anchored proteins to glycolipid-enriched membrane subdomains during transport to the apical cell surface. Cell, 1992. 68(3): p. 533-44.
45. McHugh, R.S., et al., Construction, purification, and functional incorporation on tumor cells of glycolipid-anchored human B7-1 (CD80). Proc Natl Acad Sci U S A, 1995. 92(17): p. 8059-63.
46. Hooper, N.M. and A.J. Turner, Ectoenzymes of the kidney microvillar membrane. Differential solubilization by detergents can predict a glycosyl- phosphatidylinositol membrane anchor. Biochem J, 1988. 250(3): p. 865-9.
47. Hooper, N.M. and A. Bashir, Glycosyl-phosphatidylinositol-anchored membrane proteins can be distinguished from transmembrane polypeptide-anchored proteins by differential solubilization and temperature-induced phase separation in Triton X-114. Biochem J, 1991. 280 (Pt 3): p. 745-51.
48. Eisenhaber, B., et al., Enzymes and auxiliary factors for GPI lipid anchor biosynthesis and post-translational transfer to proteins. Bioessays, 2003. 25(4): p. 367-85.
49. van Die, I., et al., Glycosylation in lepidopteran insect cells: identification of a beta 1-->4-N-acetylgalactosaminyltransferase involved in the synthesis of complex-type oligosaccharide chains. Glycobiology, 1996. 6(2): p. 157-64.
50. Delahunty, M.D., et al., Uncleaved signals for glycosylphosphatidylinositol anchoring cause retention of precursor proteins in the endoplasmic reticulum. J Biol Chem, 1993. 268(16): p. 12017-27.
51. Davies, A. and B.P. Morgan, Expression of the glycosylphosphatidylinositol-linked complement- inhibiting protein CD59 antigen in insect cells using a baculovirus vector. Biochem J, 1993. 295(Pt 3): p. 889-96.
52. Kennard, M.L., et al., Expression of cell surface GPI-anchored human p97 in baculovirus infected insect cells. Biotech. Bioeng., 1997. 55: p. 41-53.
53. Martinez, A.P., et al., The roles of the glycosylphosphatidylinositol anchor on the production and immunogenicity of recombinant ookinete surface antigen Pbs21 of Plasmodium berghei when prepared in a baculovirus expression system. Parasite Immunol, 2000. 22(10): p. 493-500.
54. Pan, W., et al., Vaccine candidate MSP-1 from Plasmodium falciparum: a redesigned 4917 bp polynucleotide enables synthesis and isolation of full-length protein from Escherichia coli and mammalian cells. Nucleic Acids Res, 1999. 27(4): p. 1094-103.
55. Tine, J.A., et al., NYVAC-Pf7: a poxvirus-vectored, multiantigen, multistage vaccine candidate for Plasmodium falciparum malaria. Infect Immun, 1996. 64(9): p. 3833-44.
56. Moran, P. and I.W. Caras, Requirements for glycosylphosphatidylinositol attachment are similar but not identical in mammalian cells and parasitic protozoa. J Cell Biol, 1994. 125(2): p. 333-43.
57. Burghaus, P.A., et al., Analysis of recombinant merozoite surface protein-1 of Plasmodium falciparum expressed in mammalian cells. Mol Biochem Parasitol, 1999. 104(2): p. 171-83.
58. Kedees, M.H., et al., Processing and localisation of a GPI-anchored Plasmodium falciparum surface protein expressed by the baculovirus system. Eur J Cell Biol, 2000. 79(1): p. 52-61.
59. Hegedus, D.D., et al., Differences in the expression and localization of human melanotransferrin in lepidopteran and dipteran insect cell lines. Protein Expr Purif, 1999. 15(3): p. 296-307.
60. Roberts, W.L., et al., Structural characterization of the glycoinositol phospholipid membrane anchor of human erythrocyte acetylcholinesterase by fast atom bombardment mass spectrometry. J Biol Chem, 1988. 263(35): p. 18776-84.
61. Naik, R.S., E.A. Davidson, and D.C. Gowda, Developmental stage-specific biosynthesis of glycosylphosphatidylinositol anchors in intraerythrocytic Plasmodium falciparum and its inhibition in a novel manner by mannosamine. J Biol Chem, 2000a. 275(32): p. 24506-11.
62. Gowda, D.C., Structure and activity of glycosylphosphatidylinositol anchors of plasmodium falciparum. Microbes and infection, 2002. 4: p. 983-990.
63. Howell, S., et al., A cleavable N-terminal signal peptide is not a prerequisite for the biosynthesis of glycosylphosphatidylinositol-anchored proteins. J Biol Chem, 1994. 269(25): p. 16993-6.
64. Altmann, F. et al., Insect cells as hosts for the expression of recombinant glycoproteins. Glycoconj J 1999. 16(2): p.109-123.
65. Wilson, I.B. et al., Core alpha 1,3-fucose is a key part of the epitope recognized by antibodies reacting against plant N-linked oligosaccharides and is present in a wide variety of plant extracts. Glycobiology 1998. 8(7): p. 651-661.
66. Darko, C.A., Angov, E., Collins, W.E. et al. The clinical-grade 42-kilodalton fragment of merozoite surface protein 1 of Plasmodium falciparum strain FVO expressed in Escherichia coli protects Aotus nancymai against challenge with homologous erythrocytic-stage parasites. Infect Immun 2005 ;73(1):287-297.
67. Stowers, A.W., Cioce, V., Shimp, R.L. et al. Efficacy of two alternate vaccines based on plasmodium falciparum merozoite surface protein 1 in an Aotus challenge Trial. Infect Immun 2001;69(3):1536-1546.
68. John, C.C., O'Donnell, R.A., Sumba, P.O. et al. Evidence that invasion-inhibitory antibodies specific for the 19-kDa fragment of merozoite surface protein- 1 (MSP- 119) can play a protective role against blood-stage Plasmodium falciparum infection in individuals in a malaria endemic area of Africa. J Immunol 2004; 173(1):666-672.
69. Slack, J., et al., Subclass restriction of murine antibodies. II. The IgG plaque-forming cell response to thymus-independent type 1 and type 2 antigens in normal mice and mice expressing an X-linked immunodeficiency. J Exp Med, 1980. 151(4): p. 853-62.
70. Taylor-Robinson, A.W., et al., The role of TH1 and TH2 cells in a rodent malaria infection. Science, 1993. 260(5116): p. 1931-4.
71. Long, C.A., et al., Immunity to erythrocytic stages of malarial parasites. Am J Trop Med Hyg, 1994. 50(4 Suppl): p. 27-32.
72. Udhayakumar, V., et al., Identification of T and B cell epitopes recognized by humans in the C-terminal 42-kDa domain of the Plasmodium falciparum merozoite surface protein (MSP)-1. J Immunol, 1995. 154(11): p. 6022-30.
73. Egan, A., et al., Characterization of human T- and B-cell epitopes in the C terminus of Plasmodium falciparum merozoite surface protein 1: evidence for poor T-cell recognition of polypeptides with numerous disulfide bonds. Infect Immun, 1997. 65(8): p. 3024-31.
74. Dutta, S., et al., Purification, characterization, and immunogenicity of a disulfide cross- linked Plasmodium vivax vaccine candidate antigen, merozoite surface protein 1, expressed in Escherichia coli. Infect Immun, 2001. 69(9): p. 5464-70.
75. Falanga, P.B., et al., Isotypic pattern of the polyclonal B cell response during primary infection by Plasmodium chabaudi and in immune-protected mice. Eur J Immunol, 1987. 17(5): p. 599-603.
76. White, W.I., C.B. Evans, and D.W. Taylor, Antimalarial antibodies of the immunoglobulin G2a isotype modulate parasitemias in mice infected with Plasmodium yoelii. Infect Immun, 1991. 59(10): p. 3547-54.
77. Von der Weid, T., et al., The immune response to Plasmodium chabaudi malaria in interleukin-4-deficient mice. Eur J Immunol, 1994. 24(10): p. 2285-93.
78. Wunderlich, G., I.C. Moura, and H.A. del Portillo, Genetic immunization of BALB/c mice with a plasmid bearing the gene coding for a hybrid merozoite surface protein 1-hepatitis B virus surface protein fusion protects mice against lethal Plasmodium chabaudi chabaudi PC1 infection. Infect Immun, 2000. 68(10): p. 5839-45.
79. Waki, S., et al., Interferon-gamma and the induction of protective IgG2a antibodies in non-lethal Plasmodium berghei infections of mice. Parasite Immunol, 1995.17(10): p. 503-8.
80. Smith, E.C. and A.W. Taylor-Robinson, Parasite-specific immunoglobulin isotypes during lethal and non-lethal murine malaria infections. Parasitol Res, 2003. 89(1): p. 26-33.
81. Perlmutter, R.M., et al., Subclass restriction of murine anti-carbohydrate antibodies. J Immunol, 1978. 121(2): p. 566-72.
82. Rosenberg, Y.J. and J.M. Chiller, Ability of antigen-specific helper cells to effect a class-restricted increase in total Ig-secreting cells in spleens after immunization with the antigen. J Exp Med, 1979. 150(3): p. 517-30.
83. Bento, C.A., et al., Glycoinositolphospholipids purified from Trypanosoma cruzi stimulate Ig production in vitro. J Immunol, 1996. 157(11): p. 4996-5001.
84. Ropert, C. and R.T. Gazzinelli, Signaling of immune system cells by glycosylphosphatidylinositol (GPI) anchor and related structures derived from parasitic protozoa. Curr Opin Microbiol, 2000. 3(4): p. 395-403.
85. Joyce, S., et al., Natural ligand of mouse CD1d1: cellular glycosylphosphatidylinositol. Science, 1998. 279(5356): p. 1541-4.
86. Molano, A., et al., Cutting edge: the IgG response to the circumsporozoite protein is MHC class II-dependent and CD1d-independent: exploring the role of GPIs in NK T cell activation and antimalarial responses. J Immunol, 2000. 164(10): p. 5005-9.
87. Procopio, D.O., et al., Glycosylphosphatidylinositol-anchored mucin-like glycoproteins from Trypanosoma cruzi bind to CD1d but do not elicit dominant innate or adaptive immune responses via the CD1d/NKT cell pathway. J Immunol, 2002. 169(7): p. 3926-33.
88. Duthie, M.S., et al., During Trypanosoma cruzi infection CD1d-restricted NK T cells limit parasitemia and augment the antibody response to a glycophosphoinositol- modified surface protein. Infect Immun, 2002. 70(1):p. 36-48.
89. Campos, M.A., et al., Activation of Toll-like receptor-2 by glycosylphosphatidylinositol anchors from a protozoan parasite. J Immunol, 2001. 167(1): p. 416-23.
90. Almeida, I.C. and R.T. Gazzinelli, Proinflammatory activity of glycosylphosphatidylinositol anchors derived from Trypanosoma cruzi: structural and functional analyses. J Leukoc Biol, 2001. 70(4): p. 467-77.
91. Link, C., et al., The Toll-like receptor ligand MALP-2 stimulates dendritic cell maturation and modulates proteasome composition and activity. Eur J Immunol, 2004. 34(3): p. 899-907.
92. Lien, E. and D.T. Golenbock, Adjuvants and their signaling pathways: beyond TLRs. Nat Immunol, 2003. 4(12): p. 1162-4.
93. Coutelier, J.P., et al., IgG2a restriction of murine antibodies elicited by viral infections. J Exp Med, 1987. 165(1): p. 64-9.
94. Markine-Goriaynoff, D., et al., IFN-gamma-independent IgG2a production in mice infected with viruses and parasites. Int Immunol, 2000. 12(2): p. 223-30.
95. Klaus, G.G., et al., Activation of mouse complement by different classes of mouse antibody. Immunology, 1979. 38(4): p. 687-95.
96. Kipps, T.J., et al., Importance of immunoglobulin isotype in human antibody-dependent, cell-mediated cytotoxicity directed by murine monoclonal antibodies. J Exp Med, 1985. 161(1):p. 1-17.
97. Heusser, C.H., C.L. Anderson, and H.M. Grey, Receptors for IgG: subclass specificity of receptors on different mouse cell types and the definition of two distinct receptors on a macrophage cell line. J Exp Med, 1977. 145(5): p. 1316-27.
98. Snapper, C.M. and W.E. Paul, Interferon-gamma and B cell stimulatory factor-1 reciprocally regulate Ig isotype production. Science, 1987. 236(4804): p. 944-7.
99. Finkelman, F.D., et al., IFN-gamma regulates the isotypes of Ig secreted during in vivo humoral immune responses. J Immunol, 1988. 140(4): p. 1022-7.
100. Rubinstein, L.J. and K.E. Stein, Murine immune response to the Neisseria meningitidis group C capsular polysaccharide. I. Ontogeny. J Immunol, 1988. 141(12): p. 4352-6.
101. Grey, H.M., J.W. Hirst, and M. Cohn, A new mouse immunoglobulin: IgG3. J Exp Med, 1971. 133(2): p. 289-304.
102. Balkovic, E.S., J.A. Florack, and H.R. Six, Immunoglobulin G subclass antibody responses of mice to influenza virus antigens given in different forms. Antiviral Res, 1987. 8(3): p. 151-60.
103. Greenspan, N.S. and L.J. Cooper, Intermolecular cooperativity: a clue to why mice have IgG3? Immunol Today, 1992. 13(5): p. 164-8.
104. Briles, D.E., et al., Mouse Igg3 antibodies are highly protective against infection with Streptococcus pneumoniae. Nature, 1981. 294(5836): p. 88-90.
105. McLay, J., et al., Gamma 3 gene-disrupted mice selectively deficient in the dominant IgG subclass made to bacterial polysaccharides. II. Increased susceptibility to fatal pneumococcal sepsis due to absence of anti-polysaccharide IgG3 is corrected by induction of anti-polysaccharide IgG1. J Immunol, 2002. 168(7): p. 3437-43.
106. Han, Y., M.H. Riesselman, and J.E. Cutler, Protection against candidiasis by an immunoglobulin G3 (IgG3) monoclonal antibody specific for the same mannotriose as an IgM protective antibody. Infect Immun, 2000. 68(3): p. 1649-54.
107. Gavin, A.L., et al., Identification of the mouse IgG3 receptor: implications for antibody effector function at the interface between innate and adaptive immunity. J Immunol, 1998. 160(1): p. 20-3.
108. Barnes, N., et al., FcgammaRl-deficient mice show multiple alterations to inflammatory and immune responses. Immunity, 2002. 16(3): p. 379-89.
109. Shparago, N., et al., IL-10 selectively regulates murine Ig isotype switching. Int Immunol, 1996. 8(5): p. 781-90.
110. Snapper, C.M. and J.J. Mond, A model for induction of T cell-independent humoral immunity in response to polysaccharide antigens. J Immunol, 1996.157(6): p. 2229-33.
111. Thelu, J., et al., Development of natural immunity in Plasmodium falciparum malaria: study of antibody response by Western immunoblotting. J Clin Microbiol, 1991. 29(3): p. 510-8.
112. Bouharoun-Tayoun, H. and P. Druilhe, Plasmodium falciparum malaria: evidence for an isotype imbalance which may be responsible for delayed acquisition of protective immunity. Infect Immun, 1992. 60(4): p. 1473-81.
113. Sarthou, J.L., et al., Prognostic value of anti-Plasmodium falciparum-specific immunoglobulin G3, cytokines, and their soluble receptors in West African patients with severe malaria. Infect Immun, 1997. 65(8): p. 3271-6.
114. Oeuvray, C., et al., Cytophilic immunoglobulin responses to Plasmodium falciparum glutamate-rich protein are correlated with protection against clinical malaria in Dielmo, Senegal. Infect Immun, 2000. 68(5): p. 2617-20.
115. Perlmann, P. and M. Troye-Blomberg, Malaria blood-stage infection and its control by the immune system. Folia Biol (Praha), 2000. 46(6): p. 210-8.
116. Yount, W.J., et al., Studies on human antibodies. VI. Selective variations in subgroup composition and genetic markers. J Exp Med, 1968. 127(3): p. 633-46.
117. Makela, O., et al., Isotype concentrations of human antibodies to Haemophilus influenzae type b polysaccharide (Hib) in young adults immunized with the polysaccharide as such or conjugated to a protein (diphtheria toxoid). J Immunol, 1987. 139(6): p. 1999-2004.
118. Ferrante, A., L.J. Beard, and R.G. Feldman, IgG subclass distribution of antibodies to bacterial and viral antigens. Pediatr Infect Dis J, 1990. 9(8 Suppl): p. S16-24.
119. Stein, K.E., Thymus-independent and thymus-dependent responses to polysaccharide antigens. J Infect Dis, 1992. 165 Suppl 1: p. S49-52.
120. Der Balian, G.P., et al., Subclass restriction of murine antibodies. III. Antigens that stimulate IgG3 in mice stimulate IgG2c in rats. J Exp Med, 1980. 152(1): p. 209-18.
121. Del Portillo et al., Primary structure of the merozoite surface antigen 1 of Plasmodium vivax neveals sequences conserved between different Plasmodium species. Proc Natl Acad Sci, 1991. 88(9): p4030-4.

## Claims

1. Purified polypeptide comprising or consisting of a C-terminus MSP1 antigen from *Plasmodium* carrying a Glycosyl-phosphatidyl-inositol group (GPI).

2. Purified polypeptide according to claim 1, wherein said MSP1 antigen is MSP1-19 peptide from *Plasmodium.*

3. Purified polypeptide according to claim 1, wherein said C-terminus MSP1 antigen is a polypeptide fragment comprising from 10 to 200 amino acids, and preferably from 10 to 150 amino acids.

4. Purified polypeptide according to any one of claims 1 to 3, wherein said MSP1 antigen is a *Plasmodium falciparum* antigen.

5. Purified polypeptide according to any one of claims 1 to 4, which is a recombinant polypeptide recovered from the plasma membrane of a eucaryotic cell where it is anchored through GPI.

6. Purified polypeptide according to claim 5, wherein said MSP1-19 peptide has the following amino-acid sequence:
VTHESYQELVKKLEAFEDAVLTGYEFNTIISKLIEGKFQDMLNISQHQCVKK QCPENSGCFRHLDEREECKCLLNYKQEGDKCVENPNPTCNENNGGCDA DAKCTEEDSGSNGKKITCECTKPDSYPLFDGIFCSGIFSS (SEQ ID NO: 1).

7. Purified polypeptide according to any one of Claims 1 to 5, which is recognized by conformation-dependent antibodies directed against the MSP1-19 peptide.

8. Purified polypeptide according to any one of claims 5 to 7, wherein said MSP1-19 peptide reproduces the disulphide bonded double EGF-like domain structure of native MSP1 p19.

9. Purified polypeptide according to any one of Claims 1 to 8, which is expressed in insect cells, and preferably in a baculovirus / insect cell expression system.

10. Purified polypeptide according to any one of Claim 1 to 9, wherein the MSP1-19 peptide is expressed with a His-tag and has the following sequence:
VTHESYQELVKKLEAFEDAVLTGYEFNTIISKLIEGKFQDMLNISQHQCVKK QCPENSGCFRHLDEREECKCLLNYKQEGDKCVENPNPTCNENNGGCDA DAKCTEEDSGSNGKKITCECTKPDSYPLFDGIFCSHHHHHHGIFSS (SEQ ID NO: 2).

11. A purified variant having at least 80% identity with the purified polypeptide according to any one of claims 1 to 10 resulting from an amino acid sequence substitution deletion or addition of at least one amino acid, with the proviso that it retains the GPI group and the immunogenic properties of said purified polypeptide.

12. The variant according to claim 11, wherein said variant has less than 200 amino acids, preferably less than 50 amino acids.

13. An immunogenic composition, wherein said composition comprises a purified polypeptide or variant according to any one of claims 1 to 12, and a pharmaceutically acceptable vehicule.

14. The composition according to claim 13, wherein said composition is devoid of additional compounds known as adjuvant of the immune response.

15. A vaccine against a *Plasmodium* infection, wherein said vaccine comprises or consists of an active principle which is the purified polypeptide or variant according to any one of claims 1 to 12.

16. The vaccine according to claim 15, wherein said vaccine is devoid of additional compounds known as adjuvant of the immune response.

17. Vaccine according to Claim 15 or 16, which further comprises polymer additives.

18. Vaccine according to any one of Claims 15 to 17, wherein a further antigen of *Plasmodium* is added as active principle.

19. Vaccine according to any one of claims 15 to 18 for use against infection by *Plasmodium falciparum.*

20. A nucleic acid molecule which encodes the amino acid sequence of the purified polypeptide of Claim 10 or the amino acid sequence of a variant of claim 11 or 12.

21. A nucleic acid molecule which encodes the amino acid sequence of the purified polypeptide of Claim 10 or the amino acid sequence of a variant of claims 11 or 12, which is linked to a nucleic acid molecule which encodes a signal peptide having or comprising the following sequence MKIIFFLCSFLFFIINTQC (SEQ lD NO: 3) and a nucleic acid molecule which encodes an anchor peptide, that signal for GPI addition, having or comprising the following sequence SSNFNLGISFLLILMLILYSFI (SEQ ID NO: 4).

22. A nucleic acid molecule which encodes a purified polypeptide according to any one of Claims 1 to 10 or a variant of Claim 11 or 12, which has a nucleotide sequence which is optimised for codon usage appropriate in baculovirus expression vector.

23. A nucleic acid molecule according to any one of claims 20 to 22, which has the following nucleotide sequence:
- the nucleotide sequence Sig1, of SEQ ID No. 7, and corresponding to the sequence
CCCGGATCCGAAAATGAAGATCATATTCTTTTTGTGTTCGTTCCTCTTC
- the nucleotide sequence Sig2, of SEQ ID No. 8, and corresponding to the sequence
TCTCGTGGGTCACACATTGCGTGTTGATGATGAAGAAGAGGAACGAAC
- the nucleotide sequence Sig3, of SEQ ID No. 9, and corresponding to the sequence
GTGTGACCCACGAGAGCTACCAAGAGCTCGTCAAGAAACTGGAGGCC TTC
- the nucleotide sequence Sig4, of SEQ ID No. 10, and corresponding to the sequence
CCCGAATTCGTAGCCGGTCAACACCGCGTCCTCGAAAGGCCTCCAGT TTC
- the nucleotide sequence Bac1, of SEQ ID No. 11, and corresponding to the sequence
GGGGAATTCAACATCTCGCAGCACCAATGCGTGAAAAAACAATGTCCC GAGAACTCTGGCTGTTTCAGACACTTGGACGAGAGA
- the nucleotide sequence Bac2, of SEQ ID No. 12, and corresponding to the sequence
ACAGGTCGGGTTGGGGTTCTCCACGCACTTGTCGCCCTCCTGTTTGTA GTTCAGCAGACATTTACACTCCTCTCTCTCGTCCAAGTG
- the nucleotide sequence Bac3 SEQ ID No. 13, and corresponding to the sequence
CCCAACCCGACCTGTAACGAGAACAACGGCGGCTGTGACGCCGACG CCAAATGCACCGAGGAGGACTCGGGCAGCAACGGCAAG
- the nucleotide sequence Bac4, of SEQ ID No. 14, and corresponding to the sequence
AGAGGAGCTGCAGAAGATGCCGTCGAACAGCGGGTACGAGTCGGGT TTGGTACACTCACACGTGATTTTCTTGCCGTTGCTGCC
- the nucleotide sequence Bac5, of SEQ ID No. 15, and corresponding to the sequence
TTCTGCAGCTCCTCTAACTTCTTGGGCATCTCGTTCTTGTTGATCCTCA TGTTGATCTTGTACAGCTTCATTTAATAAAGATCTCCC
- the nucleotide sequence Bac6, of SEQ ID No. 16, and corresponding to the sequence GGGAGATCTTTATTAAATGAAGC
- the nucleotide sequence Bac7, of SEQ ID No. 17, and corresponding to the sequence GGGGAATTCAACATCTCGCAGC
- the nucleotide sequence Bac8, of SEQ ID No. 18, and corresponding to the sequence
GCCCAAAGATCTTTATTAGCTGCAGAAGATGCCGTCGAACAGCGG

24. An expression vector for the expression of a purified polypeptide according to any one of claims 1 to 10, comprising a nucleic acid molecule according to any one of Claims 20 to 23.

25. The expression vector according to claim 24 for the expression of purified polypeptide, wherein said expression vector is a baculovirus expression vector.

26. An expression system for the expression of purified polypeptide according to any one of Claims 1 to 10 or a variant of Claim 11 or 12, wherein said expression system comprises a baculovirus expression vector according to anyone of Claim 24 or 25 and insect cells.

27. Polypeptide according to any one of the claims 1 to 10, further **characterized in that** it has the amino acid sequence of SEQ ID NO 6.

28. Polypeptide according to any one of the claims 1 to 10, further **characterized in that** it has the amino acid sequence of SEQ ID NO 20.

29. A polynucleotide according to any one of claims 20 to 22, further **characterized in that** is has the sequence of SEQ ID NO 5.

30. A polynucleotide according to any one of claims 20 to 22, further **characterized in that** is has the sequence of SEQ ID NO 19.

31. The recombinant baculovirus PfMSP1p19/His/GPI deposited at the C.N.C.M. on November 10, 2005 under the accession number CNCM I-3515.

32. The recombinant baculovirus PvMSP1p19/His/GPI deposited at the C.N.C.M. on November 10, 2005 under the accession number CNCM I-3516.

33. A polynucleotide which consists of the nucleic acid fragment of MSP1p19 contained in PfMSP1p19/His/GPl (CNCM I-3515) or in PvMSP1p19/His/GPI (CNCM I-3516).
